(19) Europäisches Patentamt
European Patent Office
Office européen des brevets

(11) **EP 4 097 715 B1**

(12) **EUROPEAN PATENT SPECIFICATION**

(45) Date of publication and mention
of the grant of the patent:
**30.08.2023 Bulletin 2023/35**

(21) Application number: **21732667.7**

(22) Date of filing: **19.05.2021**

(51) International Patent Classification (IPC):
*G10K 11/18* (2006.01)      *A61B 8/00* (2006.01)
*G01S 15/00* (2020.01)      *H02J 50/15* (2016.01)
*H02J 50/80* (2016.01)      *H02J 50/90* (2016.01)
*H04B 11/00* (2006.01)      *A61B 5/00* (2006.01)
*H02J 50/40* (2016.01)

(52) Cooperative Patent Classification (CPC):
**G10K 11/18; H02J 50/15; H02J 50/402;**
**H02J 50/90;** A61B 34/72; H02J 2310/23;
Y02E 10/30

(86) International application number:
**PCT/US2021/033053**

(87) International publication number:
**WO 2021/242580 (02.12.2021 Gazette 2021/48)**

(54) **MITIGATING MECHANICAL WAVE SIGNAL ABSORPTION ACCROSS MULTIPLE ENERGY-HARVESTING DEVICES**

VERRINGERUNG DER ABSORPTION EINER MECHANISCHEN WELLEN ÜBER MEHRERE ENERGIE-GEWINNENDE VORRICHTUNGEN

MITIGATION D'ABSORPTION D'UN ONDE MECANIQUE È TRAVERS D'UNE PLURALITÉ DE DISPOSITIFS DE PRÉLEVEMENT D'ÉNERGIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.05.2020 US 202063032023 P**

(43) Date of publication of application:
**07.12.2022 Bulletin 2022/49**

(73) Proprietor: **CBN Nano Technologies Inc.**
**Ottawa, ON K1Z 1A1 (CA)**

(72) Inventor: **HOGG, Tad**
**Mountain View, California 94040 (US)**

(74) Representative: **sgb europe**
**Lechnerstraße 25a**
**82067 Ebenhausen (DE)**

(56) References cited:
**WO-A2-2015/187742      US-B1- 10 024 950**

• **STEFANUS A WIRDATMADJA ET AL: "Wireless Optogenetic Nanonetworks: Device Model and Charging Protocols", ARXIV.ORG, CORNELL UNIVERSITY LIBRARY, 201 OLIN LIBRARY CORNELL UNIVERSITY ITHACA, NY 14853, 20 June 2017 (2017-06-20), XP080771245,**
• **MISRA SUDIP ET AL: "Long-Term Alleviation of Parkinsonian Resting Tremor Using Wireless Optogenetic Nanonetworks", IEEE TRANSACTIONS ON NANOBIOSCIENCE, IEEE SERVICE CENTER, PISCATAWAY, NY, US, vol. 19, no. 3, 12 March 2020 (2020-03-12) , pages 403-409, XP011796782, ISSN: 1536-1241, DOI: 10.1109/TNB.2020.2979781 [retrieved on 2020-06-30]**
• **MOHAMMAD MERAJ GHANBARI ET AL: "A Sub-mm^3 Ultrasonic Freefloating Implant for Multi-mote Neural Recording", ARXIV.ORG, CORNELL UNIVERSITY LIBRARY, 201 OLIN LIBRARY CORNELL UNIVERSITY ITHACA, NY 14853, 19 May 2019 (2019-05-19), XP081437269,**

- **TAD HOGG ET AL: "Acoustic communication for medical nanorobots", NANO COMMUNICATION NETWORKS, ELSEVIER, AMSTERDAM, NL, vol. 3, no. 2, 25 February 2012 (2012-02-25), pages 83-102, XP028481936, ISSN: 1878-7789, DOI: 10.1016/J.NANCOM.2012.02.002 [retrieved on 2012-03-03] cited in the application**

**Description**

The present application relates to operation of large numbers of devices that absorb a mechanical wave signal such as an acoustic signal.

Remote devices may serve for a variety of uses. Signals are frequently used to power devices and/or to communicate with devices. For devices operating within a suitable transmissive material, such signals can be transmitted as mechanical waves; for liquid environments, pressure waves (referred to herein generally as "acoustic signals") are frequently employed. As examples of device operations where acoustic signals are employed, U.S. Patents 8,743,659; 8,743,660; 8,755,252; 8,760,972; 8,787,115; 8,837,258; and 10,024,950 describe power and/or communication signals transmitted to, from, and/or between devices which can be micro-scale or nano-scale in size. In one example of a suitable application, such devices can operate inside the body of an organism for medical purposes.

Document "Wireless Optogenetic Nanonetworks: Device Model and Charging Protocols", by Stefanus A. Wirdatmadja et al., ARXIV.ORG, Cornell University Library, 201 Olin Library Cornell University Ithaca, NY 14853, 20 June 2017, discloses the concept of wireless optogenetic nanonetworking devices (WiOptND) that could address the problem of long-term deployment and also target single neuron stimulation. The WiOptND is equipped with a miniature LED that is able to stimulate the genetically engineered neuron and at the same time harvest energy from ultrasonic vibrations. The document investigates the behavior of light propagation in the brain tissue. A number of charging protocols are proposed in order to minimize the quantity of energy required for charging.

Document MISRA SUDIP ET AL: "Long-Term Alleviation of Parkinsonian Resting Tremor Using Wireless Optogenetic Nanonetworks'", IEEE TRANSACTIONS ON NANOBIOSCIENCE, IEEE SERVICE CENTER, PISCATAWAY, NY, US, vol. 19, no. 3, 12 March 2020 (2020-03-12), pages 403-409, ISSN: 1536-1241, DOI 10.1109/TNB.2020.2979781 discusses wireless optogenetic networks of neural dusts to provide a long-term solution for the alleviation of resting tumor. Interfaced with neural dusts, each of the subthalamic nucleus neurons can be controlled and stimulated by ultrasonic waves which are transmitted from a single/multiple subdural transducer(s) that are placed in the dura mater of the brain. In order to address the challenging tasks of charging and addressing each of the neural dusts, a Single Time Instant Addressing Protocol is proposed, which outperforms the state of the art parallel charging protocol. The basic idea of the protocol is that it selects most frequently occurring spike patterns in a single time instant and assigns the pattern with an ultrasonic frequency.

Document MOHAMMAD MERAJ GHANBARI ET AL: "A Sub-mm'3 Ultrasonic Freefloating Implant for Multimote Neural Recording", ARXIV.ORG, CORNELL UNIVERSITY LIBRARY, 201 OLIN LIBRARY CORNELL UNIVERSITY ITHACA, NY 14853, 19 May 2019 (2019-05-19), , discloses a sub-mm$^3$ ultrasonic free-floating implant for multi-mote neural recording. The implant uses a single ultrasound link for wireless power harvesting and analog data back telemetry. Simultaneous power-up and communication with two free-floating motes is demonstrated without requiring a specialized, e.g., beamformed, external transducer.

Document WO 2015/187742 A2 describes buoyant sensor networks, comprising floating buoys with sensors and energy harvesting capabilities. The buoys can control their buoyancy and motion and can organize communication in a distributed fashion. Some buoys may have tethered underwater vehicles with a smart spooling system that allows the vehicles to dive deep underwater while remaining in communication and connection with the buoys.

Document US 10 024 950 B1 describes devices and methods for communicating, powering, shaping fields, and creating fields using one or more macro-devices and one or more micro-devices, or a plurality of micro-devices. The document provides devices and methods for using acoustics to communicate between one or more macro-scale transceivers and one or more micro-devices, or between multiple micro-devices. The micro-devices may passively scatter or reflect sound from a transceiver, actively generate sound or both. Acoustic waves can also provide power to a micro-device, or may be directed to other purposes such as creating localized heating, shear, or movement. Micro-devices may act in concert to create or shape sound fields. Attenuation of a mechanical wave signal caused by many devices absorbing the signal can be mitigated by methods of operation, for example, where the properties and/or actions of devices are adjusted to reduce the absorption of the signal by devices that are located closer to a signal transmission source ("closer" generally being defined in terms of signal propagation, not necessarily merely by distance). According to the invention, a method of operating a plurality of devices, is provided. According to the invention, the devices being configured to extract energy from a mechanical wave signal, the method comprising the steps of:

distributing the devices throughout a transmissive material;
transmitting a mechanical wave signal into the transmissive material from at least one transmission source; and
adjusting the operation of devices based on their proximity to the transmission source to limit the attenuation of the mechanical wave signal, which is caused by the extraction of the mechanical wave energy by devices closer to the transmission source, as received by at least one selected location further from the transmission source.

Accordingly, a number of devices are distributed throughout a transmissive material, and a mechanical wave signal is transmitted into the transmissive material from at least one transmission source (for example, a transducer). The operation of devices can then be adjusted in order to limit the attenuation of the signal caused by those devices that are closer to the transmission source, as received by at least one selected location that is further from the transmission source. The mechanical wave signal can be used for various purposes, such as to provide power to the devices and/or to communicate to the devices, and the selected location could be a specific location in the transmissive material (for example, when operating in a biological organism, the selected location could be a specific body part of the organism) or a relative location (for example, the selected location could simply be the region in the transmissive material greater than a certain distance from the transmission source(s), or could be a particular device or group of devices that are further from the transmission source). Similarly, "proximity" to the transmission source could be treated as a relative proximity (for example, devices closer relative to other devices), could be an absolute proximity (for example, all devices within a specified distance), or proximity as determined by other factors (such as based on received signal strength, regardless of the actual physical distance from the transmission source). In the examples discussed herein, mitigation of attenuation is addressed using the example of pressure wave signals (referred to herein as "acoustic signals").

In one group of methods, the operation of the devices closer to the transmission source is adjusted to reduce their absorption of the signal while they are operating. The devices could deactivate some of the structures they use to absorb the signal, and/or could adjust such devices to reduce their absorption while they remain active (such as by limiting the range over which such structures respond to pressure variations, and/or limiting their response speed). The closer devices can cease all active absorption for coordinated periods; such periods can be coordinated, for example based on time or by a transmitted control signal. In some cases, only those devices that meet particular location criteria cease actively absorbing the signal. In one example, devices can cease (or significantly reduce) absorption only if they are located within a path between the transmission source and the selected location. Where a control signal is used to coordinate the action, such control signal can be transmitted from various locations, such as from the transmission source, or from one or more for the devices themselves. For example, a device that needs to conduct an operation requiring a strong mechanical wave signal strength could transmit a control signal for devices that are located between it and the nearest transmission source to temporarily cease operations to avoid attenuation of the signal, as received by the device transmitting the control signal.

In another group of methods (which in many cases overlaps with the above group), the operation of the devices is adjusted to reduce their absorption of the signal based on their location. As noted above, devices can cease or reduce active absorption based on their location (whether or not responsive to a control signal, such as discussed above). Where the devices have positioning capability, they can move (or avoid motion) so as to avoid a path between the transmission source and the selected location. In some methods, devices that are operated closer to the transmission source are designed to absorb less of the mechanical wave signal than devices operated further from the transmission source.

In another group of methods (which again may overlap with the methods discussed above), the mechanical wave signal is transmitted on more than one frequency, and the frequency at which the devices actively absorb the signal is adjusted to reduce attenuation; for example, they can receive on a frequency based on their proximity to the transmission source. As one example, those devices that are located closer to the transmission source can absorb a higher frequency signal than those devices located further away.

FIG. 1 is an isometric view illustrating a piston assembly that can provide one example of a component for absorbing an acoustic signal for use by a device.

FIG. 2 illustrates one example of how the response of a small-scale piston assembly such as shown in FIG. 1 can be adjusted by overlapping sheets that form an adjustable constant-force spring resisting displacement of a piston relative to a housing; attractive forces such as van der Waals attraction between the sheets provide a resistance to displacement of the piston.

FIG. 3 is a graph illustrating viscous drag factors for the piston assembly shown in FIG. 1 as a function of its displacement, compared to the value for an isolated disk moving through fluid.

FIG. 4 is a graph illustrating displacement of pistons responsive to an acoustic pressure wave, showing the position for pistons connected to three different loads.

FIG. 5A illustrates one example of how a number of pistons can be arranged on a spherical device. FIGS. 5B-5E illustrate alternative devices, where the surface of the device can be compressed to provide a piston-like action.

FIGS. 6A and 6B are graphs illustrating available signal power for devices at different distances from a transmission source as a function of frequency, for the cases of soft tissue (6A) and for lung tissue (6B).

FIG. 7 is a graph illustrating effective energy-absorption cross section for a device in soft tissue as a function of frequency, comparing the case where a device is actively absorbing the signal and the case where a device is effectively rigid.

FIGS. 8A and 8B are graphs illustrating signal attenuation, caused by a swarm of 1-micron dia. devices, at different distances from a transmission source as a function of frequency, for the cases of soft tissue (8A) and for lung tissue (8B).

FIG. 9 is a graph illustrating power attenuation as a function of number of devices for two different distances and frequencies.

FIG. 10 is a graph illustrating power attenuation as a function of distance, comparing the case where devices absorb as much of the signal as possible and the case where absorption is capped.

FIG. 11 is a graph illustrating power attenuation with distance when the signal is transmitted on two different frequencies, comparing the method where devices closer to the transmission source absorb at a higher frequency to the methods where a single frequency is used.

FIG. 12 is a graph illustrating signal power as a function of frequency comparing the method where devices in a specified region cease absorbing the signal and the method where the devices absorb regardless of location, with the results compared at two distances from the transmission source.

FIGS. 13A & 13B are graphs illustrating available power as a function of frequency for devices at three distances from the transmission source, for larger (1mm dia.) devices operating in a body of water. The number of devices for FIG. 13A is selected to have the same total volume as for the 1-micron devices illustrated in FIGS. 8A & 8B for soft tissue and lung tissue. A much smaller number of devices is used in FIG. 13B.

FIG. 14 is a graph illustrating available power vs. frequency at three distances similar to FIG. 13A, but where the power absorbed by each device is capped.

FIG. 15 is a graph comparing the power vs. frequency curves from FIG. 13A and FIG. 14 (with a different vertical scale) to show the difference in available power at 10cm distance, particularly with higher frequencies in the range considered.

FIG. 16 is a graph illustrating attenuation of signal power with distance when the signal is split into 50KHz and 100KHz components (solid lines), compared to when either frequency is used alone (broken lines).

FIG. 17 is a graph comparing attenuation with distance similarly to FIG. 16, but where the signal is split between 100KHz and 300KHz.

FIG. 18 is a graph illustrating attenuation of signal power with distance when the signal is split between three frequencies, 50KHz, 100KHz, and 300KHz.

FIG. 19 is a graph comparing available signal power with frequency at three distances (indicated by the data point shapes), for three different methods - one where devices are as shown in FIG. 5 (i.e., the same curves as in FIG. 13A), one where all devices are equipped with fewer pistons with a greater range of motion (dashed lines), and a mixed method where only devices closer to the signal source (i.e., within 3 cm) have fewer, larger-range pistons (dotted lines).

FIG. 20 is a graph similar to that of FIG. 19, but comparing the methods of all devices having more, smaller-range pistons (solid lines, same curves as FIG. 13A) and all devices having fewer, larger-range pistons (dashed lines) against a different mixed-device method; in this method (dotted lines), the devices having fewer, larger-range pistons are located further from the signal source (i.e., beyond 3 cm) than the devices having more, smaller-range pistons.

FIG. 21 is a graph comparing methods similar to those shown in FIG. 19, but where the devices having fewer pistons have the same size pistons as those with more pistons, thus having greater free internal volume. The methods shown are for all devices having more pistons (solid lines, same curves as FIG. 13A) and for those devices within 3 cm of the signal source having fewer pistons (dotted lines).

FIG. 22 is a graph comparing power vs. frequency at a 15 cm distance from the signal source for two methods, one where all devices absorb as much power as they can (dashed line), and another where the devices within 10 cm of the source do not absorb (either by ceasing active absorption or by moving away from the location between the transmission source and the 15 cm depth of measurement).

FIGS. 23A-23D illustrate some examples of methods where devices stop actively absorbing along a path leading to a desired location, responsive to a control signal.

The operation of a large number of devices together in a "swarm" can affect attenuation of acoustic signals when the devices are operating within a transmissive material. Such acoustic signals may serve various purposes, such as power transmission to devices or communication to, from, and/or between devices. In some methods, acoustic power may be employed to act on structures other than the devices themselves, such as the use of acoustic power to generate heat at a desired location, such as to kill a cancer cell in a medical patient.

One previously overlooked issue when operating large numbers of devices is the attenuation caused by active absorption of an acoustic signal by the devices. As discussed herein, such active absorption is a significant cause of attenuation, and may cause significant reduction in signal intensity at locations distant from the source that transmits the acoustic signal. Thus, methods of operating devices so as to mitigate such attenuation should be beneficial in helping to assure that sufficient signal intensity exists at desired locations in the transmissive material.

The description herein deals primarily with the examples of medical devices that operate within an organism's body, the organism providing the transmissive material; typical transmissive materials in such cases include bodily fluids, such as blood, and various tissues. However, the methods discussed herein have broader applicability for any situation where a large number of devices are operating in a transmissive material through which an acoustic signal is being transmitted. Examples include swarms of devices operating in bodies of water or storage tanks, and swarms exploring liquid-filled geological formations. Similarly, the description addressed the use of the acoustic signal to provide power to the devices in the swarm; similar methods to those discussed could also be employed for acoustic signals used for communication or other purposes.

The present discussion makes use of models to evaluate viscous drag on pistons and acoustic dissipation due to thermal and viscous effects. These simulations were modelled using Comsol Multiphysics® Acoustics Module software, available from COMSOL, Inc., One First Street, Suite 4, Los Altos, CA 94022 (https://www.comsol.com/acoustics-module).

Sound consists of longitudinal waves propagating through materials. For most biological tissues, the speed of sound and density are close to the values given in Table 1.

Table 1: Acoustic properties of tissue.

| speed of sound | $c$ | 1500 m/s |
|---|---|---|
| density | $\rho$ | $10^3$ kg/m$^3$ |
| amplitude absorption coefficient | | |
| soft tissue | $\alpha_{tissue}$ | $8.3 \times 10^{-6}$ s/m |
| lung | $\alpha_{tissue}$ | $4.7 \times 10^{-4}$ s/m |

Variations in tissue reflect, scatter and absorb sound. For the frequencies considered here, attenuation is given in Table 1 for representative low- and high-absorption tissues: acoustic pressure of frequency $f$ decreases as $\exp(-\alpha_{tissue}fx)$ over a distance x.

Additional dissipation occurs in viscous and thermal boundary layers . As an example, boundary layers for devices circulating in the bloodstream are in fluid with properties given in Table 2, where the viscous and thermal properties of the fluid around the device are taken to be close to those of water and blood plasma (Freitas, "Nanomedicine, Volume I: Basic Capabilities," Landes Bioscience, 1999). The heat capacity is at constant pressure and the ratio is of the heat capacity at constant pressure to that at constant volume.

Table 2: Viscous and thermal properties of the fluid around the device.

| dynamic viscosity | $\eta$ | $10^{-3}$ Pa s |
|---|---|---|
| bulk viscosity | $\eta_{bulk}$ | $3 \times 10^{-3}$ Pa s |
| temperature | $T_{fluid}$ | 310 K |

(continued)

| | | |
|---|---|---|
| thermal conductivity | $k_{\text{thermal}}$ | 0.6 W/m/K |
| heat capacity | $C_{\text{p}}$ | 4200 J/kg/K |
| ratio of heat capacities | $\gamma$ | 1.02 |

Ultrasound imaging typically uses a single small transducer on the skin. For powering devices that operate throughout the body, a better approach is to use multiple transducers distributed over the body. The following discussion evaluates power available to devices within about 20cm of the nearest transducer. This corresponds to multiple transducers covering the body, or at least the region of the body where devices require power. In this example, the size of the devices is much smaller than the sound wavelengths analyzed, as indicated in Table 3, where examples of sound frequencies and wavelengths are compared to examples of device size: $k = 2\pi/\lambda$ is the wave number of sound with wavelengh $\lambda = c/f$ at frequency $f$, and $r_{\text{device}} = 1\mu$m is the device's radius.

Table 3: Sound frequencies and wavelengths compared to device size.

| frequency | wavelength | $k\, r_{\text{device}}$ |
|---|---|---|
| 20kHz | 75mm | $8 \times 10^{-5}$ |
| 100kHz | 15mm | $4 \times 10^{-4}$ |
| 1000kHz | 1.5mm | $4 \times 10^{-3}$ |

Various devices could be employed to make an acoustic signal in the surrounding material useful to a device. For purposes of analysis, the following discussion employs the case of pistons used to extract power for the device from the surrounding material, to provide a numerical evaluation for one example of attenuation caused by such absorption of the signal, and the effectiveness of methods that can be employed to mitigate the effects of such absorption on transmission of the signal. Such methods should be beneficial for any situations where multiple devices are absorbing a signal, including situations where the signal is transmitted for a different purpose (i.e., communication or sensing rather than power transmission), and/or situations where different structures on the devices are employed to receive the signal.

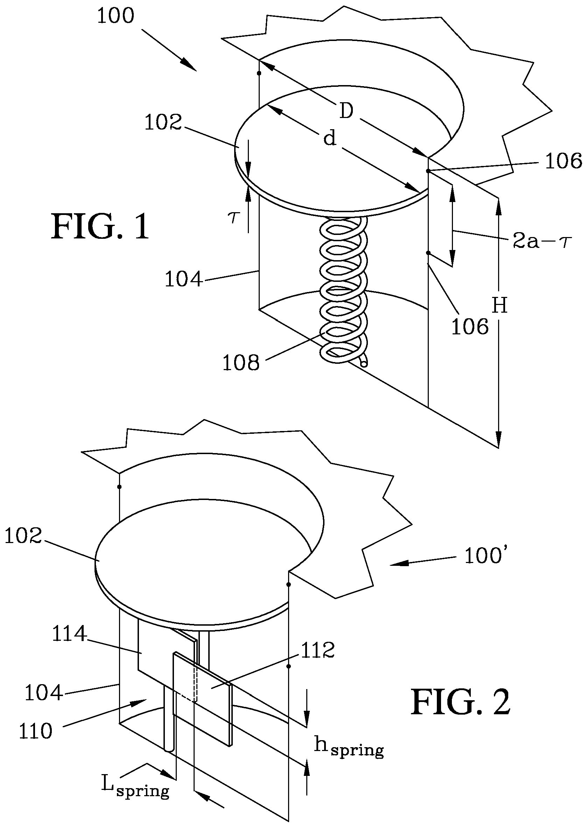

Pistons moving in response to changing pressure can extract energy from pressure waves. FIG. 1 illustrates the geometry of a piston assembly 100 having a piston 102 of diameter $d$ and thickness $\tau$, constrained to move over a limited range within a housing 104 having an internal diameter **D** and height **H** (the figures do not illustrate linkages from the piston to mechanisms powered by the piston's motion). In FIG. 1, the points 106 indicate the limits of motion of the piston 102. The distance between these points 106 is the sum of the range of motion, 2$a$, and the piston thickness $\tau$. A spring 108 under the piston 102 provides a restoring force. In the present analysis, considering devices having a radius of 1$\mu$m, the piston 102 and the housing 104 are analyzed for the dimensions set forth in Table 4, which also provides dimensions for a constant-force spring 110 as shown in FIG. 2 and discussed below.

Table 4: Geometry parameters for piston, constant-force spring and housing.

| piston | | |
|---|---|---|
| piston diameter | $d$ | 300 nm |
| piston thickness | $\tau$ | 10 nm |
| piston range of motion | 2$a$ | 200 nm |
| piston cross section area | $A = \pi(d/2)^2$ | $0.071\mu$m$^2$ |
| piston sliding area | $A_{\text{pistonsliding}} = \pi d\tau$ | $0.019\mu$m$^2$ |
| constant-force spring | | |
| perpendicular overlap | $L_{\text{spring}}$ | $\approx 35 \pm 10$ nm |
| parallel overlap | $h_{\text{spring}}$ | up to $\approx 200$ nm |
| housing | | |
| housing diameter | $D$ | 340nm |

(continued)

| housing | | |
|---|---|---|
| housing depth | $H$ | 440nm |

Sound waves are rapid pressure variations around an ambient value. The ambient pressure varies with time and position within the body (Freitas, "Nanomedicine, Volume I: Basic Capabilities," Landes Bioscience, 1999). For example, a device moving with the blood encounters decreasing pressure of about 20 kPa over about 10 seconds as it moves from arteries to veins. In addition, pressure in arteries varies by about 5 kPa during each heartbeat. These pressure variations are comparable in magnitude to safe levels of ultrasound in tissue, but change much more slowly than acoustic pressure. For example, ultrasound periods for the frequencies considered here are a few microseconds, which is much shorter than these variations in ambient pressure. In addition, device activities such as communication (Hogg and Freitas, "Acoustic communication for medical nanorobots," Nano Communication Networks, 2012) and locomotion (Hogg, "Using surface-motions for locomotion of microscopic robots in viscous fluids," J. of Micro-Bio Robotics, 2014) can produce pressure variations, generally at frequencies much higher or lower than ultrasound considered here for powering the devices. There are also small variations, of a few pascals, due to the device's changing positions when operating within a vessel (Hogg, "Stress-Based Navigation for Microscopic Robots in Viscous Fluids," Journal of Micro-Bio Robots, 2018).

Due to changes in ambient pressure, a piston with a fixed restoring force (such as provided by the spring 108 shown in FIG. 1A) would respond to acoustic pressure only when the force from the ambient pressure is close to that restoring force. Outside that range, the piston 102 would be pinned at one of its limits of motion and not collect energy. To avoid this limitation, the restoring force should adjust to changing ambient pressure. Since ambient pressure changes much more slowly than acoustic variation, the force could be adjusted based on the average measured pressure on the piston over multiple periods of the pressure wave. This measurement could use force sensors on the piston mechanism or pressure sensors elsewhere on the device surface (Freitas, "Nanomedicine, Volume I: Basic Capabilities," Landes Bioscience, 1999). Alternatively, a controller could measure the average position of the piston and adjust the restoring force to keep the average near the middle of the piston's range. As an example of the times involved, a controller could adjust the restoring force by averaging over several milliseconds to adapt to changes in ambient pressure.

One approach to compensating for ambient pressure is a spring, in which restoring force is proportional to displacement. However, the increasing force with displacement limits the piston motion and stores much of the energy from the acoustic pressure in the potential energy of the spring. Without additional mechanisms to capture that energy, the potential energy would be released back into the fluid when the acoustic pressure decreases, leading to scattering rather than absorption of acoustic energy.

Constant-force springs are a more useful approach to acoustic energy harvesting. At the nano scale, overlapping molecular devices can behave as constant-force springs due to van der Waals interactions (Cumings and Zettl, "Low-friction nanoscale linear bearing realized from multiwall carbon nanotubes," Science, 2000), (Liu, Liu et al., "Interlayer binding energy of graphite: A mesoscopic determination from deformation," Physical Review B, 20, American Physical Society, 2012). The force is in the direction of increasing overlap. This leads to the geometry shown in FIG. 2, where the constant-force spring 110 is created by a piston sheet 112 attached to the piston 102, and a housing sheet 114 that is attached to the housing 104, where the piston sheet 112 resides below the housing sheet 114 (note that the horizontal scale is exaggerated compared to FIG. 1 for purposes of illustration). The piston sheet 112 moves vertically with the piston 102, thereby changing the vertical overlap, $h_{spring}$, between the sheets (112, 114). In this configuration, van der Waals attraction draws the piston sheet 112 upwards, pushing the piston 102 against the pressure on the piston 102 from the surrounding fluid. The thick vertical lines indicate connections between the sheets (112, 114) and respective piston (102) or housing (104); these connections could be offset from the plane of overlap between the sheets (112, 114) to avoid limiting their range of relative motion. Moving the position of the housing sheet 114 horizontally within the housing 104 acts to adjust the horizontal overlap, $L_{spring}$. This adjusts the reaction force of the spring 110, since the force is proportional to the length of overlap, $L_{spring}$, between the sheets (112, 114) in the direction perpendicular to the motion:

$$F_{spring} = K_{spring} L_{spring} \qquad (1)$$

The spring constant $K_{spring}$ is 0.16 N/m for nested nanotubes (Cumings and Zettl, "Low-friction nanoscale linear bearing realized from multiwall carbon nanotubes," Science, 2000) and 0.2 N/m for graphene sheets (Liu, Liu et al., "Interlayer binding energy of graphite: A mesoscopic determination from deformation," Physical Review B, 20, American Physical Society, 2012). Sheets are convenient for this application because a horizontal change in their position, i.e.,

8

changing $L_{\text{spring}}$, allows adjusting the spring's force in response to changing ambient pressure.

For example, the force on the piston 102 due to one atmosphere of external pressure when it has the dimensions given in Table 4 is 7 nN. The constant-force spring 110 compensates for this force with an overlap $L_{\text{spring}}$ = 35 nm. This overlap is considerably less than the piston diameter **d** considered here, allowing more than enough room for the overlapping sheets (112, 114) to be adjusted within the piston housing 104.

In the geometry shown in FIG. 2, the vertical extent of each sheet (112, 114) should be at least equal to the range of piston motion (2a + **t**). This limits the piston's range to less than a third of the housing height **H.** More efficient use of housing volume is possible because horizontal overlaps several times larger than 35nm can fit within the housing diameter. Thus the constant-force spring 110 could be connected to the piston 102 via a lever: a larger overlap would increase the spring's force enough to compensate for pressure on the piston 102 while the vertical overlap of the sheets (112, 114) changes by a corresponding multiple smaller than the piston's movement. Thus, sheets having a smaller vertical extent could be employed, and the piston could move through a greater fraction of the housing, than as shown in FIG. 2. This could be useful to allow a greater range of piston motion in a given housing depth, or to reduce the housing depth required for a given range of motion, thereby reducing the housing volume and providing more space in the device for other components.

While the constant-force spring 110 shown in FIG. 2 consists of two sheets (112, 114) sliding over each other, housing 104 could contain multiple sliding sheets, with separate horizontal adjustment capability to adjust their overlaps independently, since the sheets can be made much thinner than the piston diameter. This would provide some redundancy against failures, or allow different actuators to handle adjustments over different ranges of forces. Alternatively, the force could come from combining one or more adjustable planes with nested nanotubes, which are not adjustable. In this case, the nanotubes could provide the force for the lowest ambient pressure in the body, so the adjustable planes would only need to provide forces over the range of ambient pressure above this minimum value.

For motion at the small scales considered here, drag force is proportional to the piston speed:

$$F_{\text{drag}} = k_{\text{total}} V \qquad (2)$$

with drag coefficient $k_{\text{total}}$ having contributions from friction directly due to piston motion ("internal" friction), $k_{\text{f}}$, and that arising from its use to move mechanisms within the device, $k_{\text{load}}$. The damping due to delivering power to device arises from, for example, friction of mechanical devices in the device driven by the piston's motion.

Internal friction consists of viscous drag from motion through the fluid outside the piston and sliding friction between piston and its housing:

$$k_{\text{total}} = k_{\text{f}} + k_{\text{load}}$$

$$k_{\text{f}} = k_{\text{viscous}} + k_{\text{sliding}} A_{\text{sliding}} \qquad (3)$$

where $k_{\text{viscous}}$ is drag due to viscosity, $k_{\text{sliding}} A_{\text{sliding}}$ is drag due to sliding surfaces, with $A_{\text{sliding}}$ the area of the sliding surfaces. Friction arising from motion of linkages connecting the piston motion to devices within the device are not considered separately here: instead, they are part of dissipation external to the piston included in $k_{\text{load}}$, and determine the efficiency of power use within the device.

The first contribution to $k_{\text{f}}$ in Eq. (3) arises from viscous drag. An object of size d moving at speed v through a fluid with viscosity $\eta$ at low Reynolds number experiences a drag force proportional to its diameter and the fluid viscosity (Happel and Brenner, "Low Reynolds Number Hydrodynamics," The Hague, Kluwer, 1983). Thus, a convenient characterization of this drag is the viscous drag factor g = $k_{\text{viscous}}/(d\eta)$. The value of $g$ depends on the object's shape and orientation, as well as its relation to any nearby boundaries. For instance, in unbounded fluid, a flat disk with diameter $d$ moving face-on through the fluid has $g$ = 8 (Berg, "Random Walks in Biology," Princeton University Press, 1993).

Viscous drag in this case depends on how the fluid interacts with the housing surface, in particular deviations from the no-slip boundary condition between fluids and solid surfaces (Mate, "Tribology on the Small Scale: A Bottom Up Approach to Friction, Lubrication, and Wear," OUP Oxford, 2008).

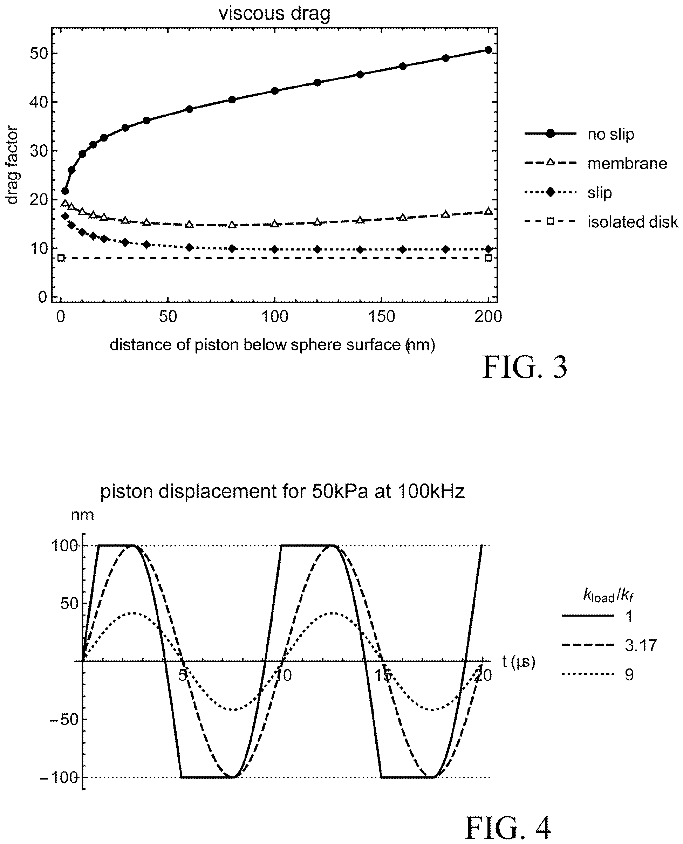

Due to this range of possibilities, the drag is evaluated here with several boundary conditions on the fluid's motion at the housing wall: 1) no-slip except for slip within 1 nm of the piston's surface due to the high shear between the stationary housing and moving piston; 2) an elastic membrane between housing and piston so that fluid speed increases linearly between housing and piston, and 3) slip along the housing wall. FIG. 3 is a graph showing the drag factor g for a piston with parameters given in Table 4 with these choices, plotting the viscous drag factor g as a function of its position below the device surface for various boundary conditions on the housing surface exposed to the fluid. For comparison, the dashed line shows the drag on an isolated disk moving face-on through the fluid far from any boundaries. Based on FIG. 3, as a worst-case estimate, the following discussion uses a relatively large value, $g$ = 45, and, for simplicity, takes $g$ to be independent of piston position.

The second contribution to $k_f$ in Eq. (3) is friction from sliding surfaces. For stiff materials, theoretical estimates of $k_{sliding}$ for atomically-flat surfaces give values somewhat less than $10^3$ kg/(m²s) (Drexler, "Nanosystems: Molecular Machinery, Manufacturing, and Computation," New York, John Wiley & Sons, 1992), (Freitas, "Nanomedicine, Volume I: Basic Capabilities," Landes Bioscience, 1999) for speeds well below the speed of sound, as is the case for piston motion. For estimating sliding friction, $k_{sliding}$ = $10^3$ kg/(m²s) is used herein.

The sliding area includes the surface at the edge of the piston 102 next to the housing 104, and the overlapping surface of the plates (112, 114) of the constant-force spring 110:

$$A_{\text{sliding}} = A_{\text{pistonsliding}} + L_{\text{spring}} h_{\text{spring}} \qquad (4)$$

The overlap area of the constant-force spring 110, $L_{\text{spring}} h_{\text{spring}}$ is less than $0.01 \mu\text{m}^2$. Thus, from Table 4, $A_{\text{sliding}} < 0.03 \mu\text{m}^2$.

With these values, $k_{\text{viscous}} = gd\eta = 1.4 \times 10^{-8}$ kg/s and $k_{\text{sliding}} A_{\text{sliding}} < 3 \times 10^{-11}$ kg/s. Thus, viscosity dominates the drag, i.e., $k_f \approx k_{\text{viscous}}$.

The above discussion estimates the drag on the piston 102 as it moves in response to acoustic pressure variation. In addition, actuators alter the overlap $L_{\text{spring}}$ of the constant-force spring 110 in response to changes in ambient pressure. The sliding drag during this adjustment is

$$F_{\text{springdrag}} = k_{\text{sliding}} \left( L_{\text{spring}} h_{\text{spring}} \right) \frac{dL_{\text{spring}}}{dt} \qquad (5)$$

An example is the changing ambient pressure during a heartbeat of around $p$ = 5 kPa in one second. This requires changing $L_{\text{spring}}$ by 2nm. Using the bound on sliding area described above, i.e, $L_{\text{spring}} h_{\text{spring}} < 0.01 \mu\text{m}^2$, the dissipation due to friction during this change is negligibly small, less than $10^{-16}$ pW.

Actuators adjusting the horizontal position of the housing sheet 114 apply forces comparable to $F = K_{\text{spring}} h_{\text{spring}}$ to change the overlap $L_{\text{spring}}$. To reduce overlap by $\Delta L_{\text{spring}}$, the actuator does work $W = F \Delta L_{\text{spring}}$. The overlap $h_{\text{spring}}$ varies with the piston's position. A typical example for the geometry of FIG. 2 is $h_{\text{spring}}$ = 100nm. Theoretical analysis indicates that, for example, electrostatic actuators could provide such forces and be small enough to adjust overlaps for the piston sizes considered here (Drexler, "Nanosystems: Molecular Machinery, Manufacturing, and Computation," New York, John Wiley & Sons, 1992). Continuing with the previous example of 5 kPa pressure change in one second, $\Delta L_{\text{spring}}$ = 2 nm, so $W$ = $4 \times 10^{-17}$ J. This energy need not be dissipated: the work done by the actuator is stored as potential energy in the position of the sheets (112, 114). This energy could be recovered as work done by the sheets (112, 114) to increase $L_{\text{spring}}$ when ambient pressure later increases. However, even if this work were entirely dissipated during the one second change in ambient pressure, it amounts to a still negligible dissipation of $10^{-5}$ pW. This potentially avoidable dissipation is much larger than that due to sliding friction described in Eq. (5), a property also seen in rotary motion of atomically precise mechanisms (Hogg, Moses et al., "Evaluating the Friction of Rotary Joints in Molecular Machines," arXiv.org, 2017).

For the position of the piston's midplane, $x$, the condition of $x$ = 0 is defined as when the piston is in the middle of its range, and the sign of $x$ is selected such that positive values mean the piston 102 is farther from the center of the device (vertically upwards in the orientation shown in FIG. 2). The maximum value of $x$ is defined as $a > 0$, so the piston's position ranges between $-a$ and $a$.

The piston 102 moves in response to forces from the applied pressure, the spring, $F_{\text{spring}}$, and damping, $F_{\text{drag}}$. The pressure is $p_{ambient} + p(t)$, where $p(t)$ is the acoustic pressure and $p_{ambient}$ is the ambient pressure in the fluid. Fluid pressure pushes inward and the spring 110 pushes outward. The piston 102 stops whenever these forces attempt to push it beyond its range of motion. Thus, unless the piston 102 is stopped at its range of motion, its position $x$ changes according to:

$$m \frac{d^2 x}{dt^2} = -F_{\text{drag}} + F_{\text{spring}} - \left( p_{\text{ambient}} + p(t) \right) A \qquad (6)$$

where $A$ is the cross-sectional area of the piston and $m$ is the piston's mass.

Viscous forces dominate the motion of objects of the size and speeds considered here (Purcell, "Life at low Reynolds number," American J. of Physics, 45, 1977). This means that the piston moves at the speed at which $F_{drag}$ balances the forces applied to the piston. That is, the piston moves at its terminal velocity in the fluid, so Eq. (6) becomes

$$k_{total} \frac{dx}{dt} = F_{\text{spring}} - \left(p_{\text{ambient}} + p(t)\right)A \tag{7}$$

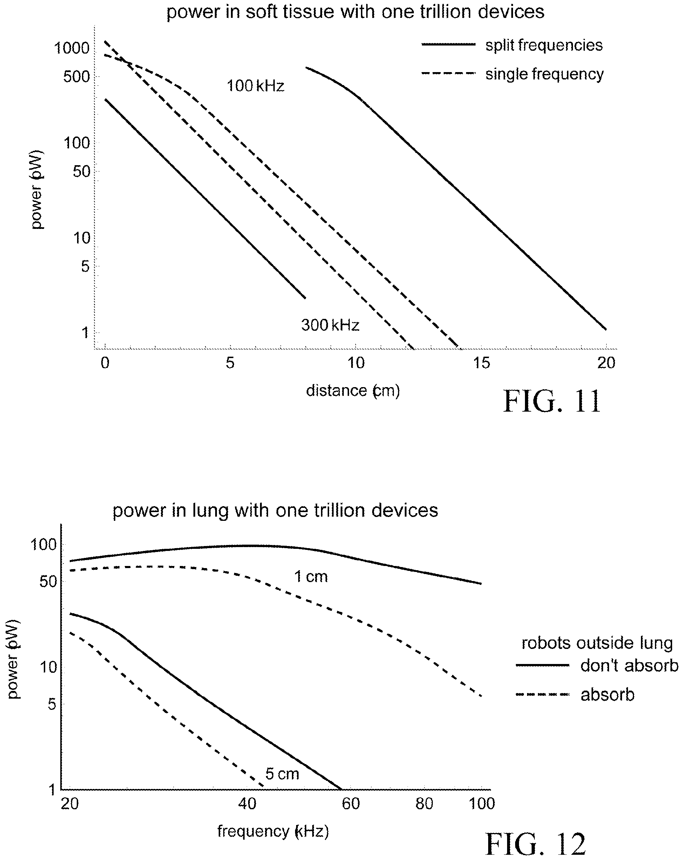

by using Eq. (2). If the piston reaches a limit to its motion, at $x = \pm a$, it remains there (i.e., $dx/dt = 0$) until the applied force changes sign. As discussed below with regard to FIG. 11, where the devices are operated in biological organisms, the ambient pressure may vary periodically, depending on the device location and/or movement. For example, for devices operating in the circulatory system, ambient pressure changes as the device moves through the circulation, experiencing high pressure in arteries and low pressure in veins. This cycle occurs over about a minute. Similarly, when in arteries, ambient pressure changes due to heart contractions, on about one second time scale, i.e., 1Hz. (Freitas, "Nanomedicine, Volume I: Basic Capabilities," Landes Bioscience, 1999)

It can be assumed that the constant-force spring adjusts to the ambient pressure, i.e., $F_{\text{spring}} = p_{\text{ambient}}A$. The origin of time is arbitrarily selected to be at the minimum acoustic pressure, so $p(t) = -p\cos(\omega t)$ for sound with angular frequency $\omega = 2\pi f$ and amplitude $p > 0$ at the device's location. With these definitions, convenient dimensionless parameters for evaluating piston motion are

$$\lambda \equiv \frac{pA}{a\omega k_f} \tag{8}$$

$$k_{\text{ratio}} \equiv \frac{k_{\text{load}}}{k_f} \tag{}$$

Defining normalized position $X = x/a$ and time as $\omega t$, Eq. (7) becomes

$$\frac{dX}{dt} = \frac{\lambda}{1+k_{\text{ratio}}} \cos(\omega t) \tag{9}$$

Viscous drag on the piston depends on its position (see FIG. 3). For simplicity, this variation is ignored in the present calculations, which instead consider the drag coefficient $k_{\text{viscous}}$, and hence $k_f$, to be independent of position, as described above. In this case, Eq. (9) gives

$$X(\tau) = \frac{\lambda}{1 + k_{\text{ratio}}} \sin(\omega t) \tag{10}$$

when the piston starts at the center of its range of motion, $X(0) = 0$.

Eq. (10) describes the complete piston motion when $A \leq 1 + k_{\text{ratio}}$. When this inequality is not satisfied, the piston spends part of each cycle stopped at the limits of its range, and Eq. (9) only holds between these stops. FIG. 4 illustrates these cases, where the motion of pistons as described in Table 4 (piston position as a function of time) is shown in response to acoustic pressure of 50kPa at 100kHz. In this case, $\lambda = 4.17$. The curves show the piston's position as a function of time with the piston connected to one of three loads: $k_{\text{load}}$ equal to $k_f$, $(\lambda - 1)k_f$ (dashed) or $9k_f$. The dashed curve is the smallest load for which Eq. (10) describes the complete motion.

As the piston moves, it dissipates energy against the drag force at the rate $F_{\text{drag}}\dot{x} = k_{\text{total}}\dot{x}^2$, from Eq. (2). Of this amount, $k_{\text{load}}\dot{x}^2$ is dissipated by the load as power available to the device in which the piston is incorporated.

When Eq. (10) describes the complete motion of the piston, the time-average power over a cycle of the acoustic pressure variation is

$$P_{\text{total}} = \frac{1}{2} A^2 p^2 \frac{1}{k_{\text{total}}} \qquad (11)$$

$$P_{\text{load}} = P_{\text{total}} \frac{k_{\text{load}}}{k_{\text{total}}}$$

From Eq. (3), the maximum $P_{\text{load}}$ occurs when $k_{\text{load}} = k_f$ (i.e., $k_{\text{ratio}} = 1$) in which case $P_{\text{load}} = P_{\text{total}}/2 = A^2 p^2/(8 k_f)$. This holds provided Eq. (10) describes the complete motion, i.e., when $\lambda \leq 2$.

If $\lambda > 2$, then the piston stops at its limit of motion during each acoustic period when $k_{\text{load}} = k_f$. The piston delivers no power while stopped. In this case, the piston delivers more power to the load for somewhat larger values of $k_{\text{load}}$. In particular, $k_{\text{ratio}} = \lambda - 1$ is the smallest load for which Eq. (10) describes the complete motion. From Eq. (11), this choice gives larger $P_{\text{load}}$ than any larger value of $k_{\text{load}}$. Numerically evaluating the motion for $k_{\text{ratio}}$ between one and $\lambda - 1$ shows the maximum $P_{\text{load}}$ occurs at $k_{\text{load}}$ slightly smaller than $\lambda - 1$. I.e., the larger power from faster piston speed while the piston is moving more than compensates for the lack of power during the short times the piston stops at its limit. However, this maximum is only slightly larger than $P_{\text{load}}$ when $k_{\text{ratio}} = \lambda - 1$: less than 3% more for $\lambda, < 5$, the range relevant for this discussion. Due to the minor benefit of smaller $k_{\text{load}}$, for simplicity the value $k_{\text{ratio}} = \lambda - 1$ is used to estimate the available power when $\lambda > 2$.

Combining these cases gives:

$$P_{\text{load}} = \begin{cases} \frac{1}{8 k_f} A^2 p^2 & \text{if } \lambda \leq 2 \\ \frac{1}{2} a\omega(Ap - a\omega k_f) & \text{if } \lambda > 2 \end{cases} \qquad (12)$$

Safety limits ultrasound intensity to about 1000W/m$^2$ for extended use (Freitas, "Nanomedicine, Volume I: Basic Capabilities," Landes Bioscience, 1999), which corresponds to pressure amplitude $p = 55$ kPa because the time-average energy flux of a plane wave is

$$\mathcal{F} = \frac{1}{2} \frac{p^2}{\rho c} \qquad (13)$$

where $p$ is the amplitude of sound pressure variation, $\rho$ is the density of the fluid and c the speed of sound.

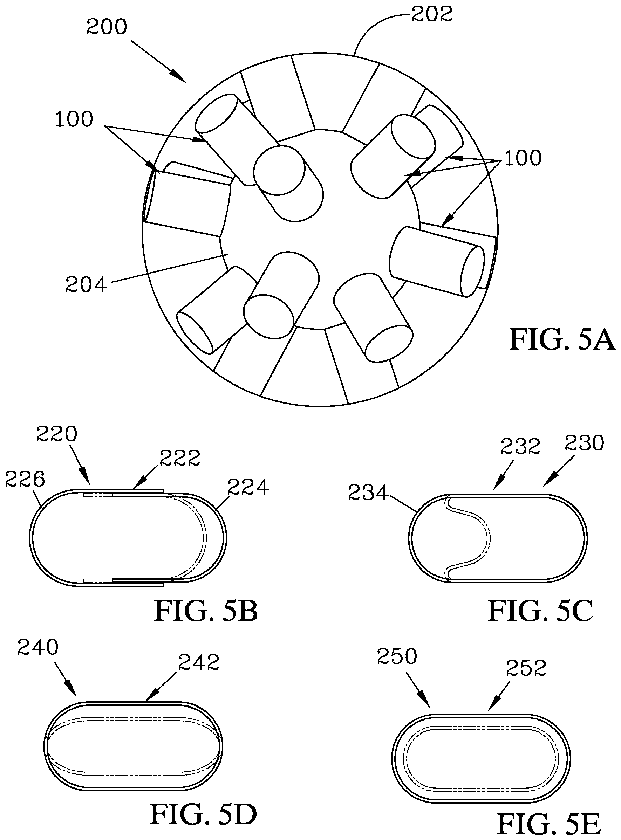

A device can use multiple pistons to extract power; one example of a device 200 with multiple piston assemblies 100 distributed relative to a spherical surface 202 is illustrated in FIG. 5A. Below the level of the piston assemblies 100, the device 200 has an internal volume represented by inner sphere 204. In some cases, it may be practical to employ a substantial portion or the entirety of the exposed surface of the device as displaceable to in response to pressure waves. FIGS. 5B-E illustrate some examples of such devices (220, 230, 240, 250). Device 220 in FIG. 5B has an outer shell 222 with two telescoping portions (224, 226) which can compress (to the configuration shown in phantom lines) and expand with respect to each other to provide an action similar to that of the piston assemblies 100. Device 230 shown in FIG. 5C employs a shell 232 that is partly flexible, having an invertible region 234 that can be pushed in by increased pressure to provide a piston-like action. Device 240 shown in FIG. 5D and device 250 shown in FIG. 5E each have compressible shells (242, 252, respectively) which could be provided by an elastic structure, overlapping plates, or similar structures. The shell 242 of the device 240 compresses preferentially in one direction, while the shell 252 of the device 250 compresses uniformly over its surface. In these devices, it may be a challenge to operate the device to reduce the amount of energy absorbed that is not re-emitted by the contraction and expansion of the shell.

For the following discussion, the device 200 is assumed to have a radius of 1 μm and the parameters given in Table 5, with each piston assembly 100 having the parameters set forth above in Table 4. The fractions in Table 5 include the piston housings 104, indicating how much of the volume and surface area of the device 200 are taken up by the piston assemblies 100. Inside the device 200, the bottoms of the piston housings 104 use 46% of the surface area indicated by the inner sphere 204. The pistons 102 themselves, i.e., the moving portion of the piston assemblies 100, use $f_{\text{surface}} = 11\%$ of the surface area of the device 200. The sound source intensity corresponds to 55kPa (Eq. (13)), which is reduced by the reflection loss to give the source pressure in the tissue next to the transducer at the transmission location. While a transducer is discussed for this example, alterative transmission sources could be employed.

Table 5: Device and signal parameters

| pistons | |
|---|---|
| duty cycle | 50% |
| number | 20 |
| fraction of device volume | 19% |
| fraction of device surface | 14% |
| transmission source | |
| intensity | 1000W/m$^2$ |
| reflection loss | 10% |
| source pressure | 50kPa |

In this scenario, pistons operate with a 50% duty cycle. This cycle could arise from changing pressure at the device's location due to adjustments to the transducer to move the sound field by half a wavelength to ensure devices are not permanently located in a pressure minimum due to interference from strong reflections, e.g., from nearby bones. The duty cycle could also arise from a device occasionally turning off power collection to enable (or simplify the design or control of) other uses for its surface that are affected by piston motions. Examples include using surface vibrations for communication (Hogg and Freitas, "Acoustic communication for medical nanorobots," Nano Communication Networks, 2012) or locomotion (Hogg, "Using surface-motions for locomotion of microscopic robots in viscous fluids," J. of Micro-Bio Robotics, 2014). in addition, piston motion alters the fluid flow near the surface, changing the pattern of stresses on the surface that the device could use to estimate its position and motion (Hogg, "Stress-Based Navigation for Microscopic Robots in Viscous Fluids," Journal of Micro-Bio Robots, 2018). Alternatively, if there is no need for this duty cycle, the results given here correspond to a device with 10 pistons operating continually, i.e., 100% duty cycle, thereby leaving more surface area for other uses, e.g., chemical sensing.

The power available to devices is considered for two situations: devices in soft tissue at various distances from the skin, and devices in lung tissue. The lung surface is about 5 cm beneath the skin, leading to some attenuation between the transducer on the skin and the surface of the lung. Furthermore, the different acoustic impedances of lung and soft tissue means only about 36% of the acoustic energy reaching the lung is transmitted into it (Freitas, "Nanomedicine, Volume I: Basic Capabilities," Landes Bioscience, 1999). In addition, the lung is partly covered by the ribs. Bone transmits only part of the sound reaching it, and the sound that does travel through bone attenuates about 20 times more rapidly than attenuation in soft tissue (Freitas, "Nanomedicine, Volume I: Basic Capabilities," Landes Bioscience, 1999). The power reaching various parts of the lung depends on their locations relative to the ribs and transducers on the skin. As an approximate accounting of these factors, acoustic energy for the lung is considered here as first attenuating through 5cm of soft tissue, after which 20% of the incident energy enters the lung.

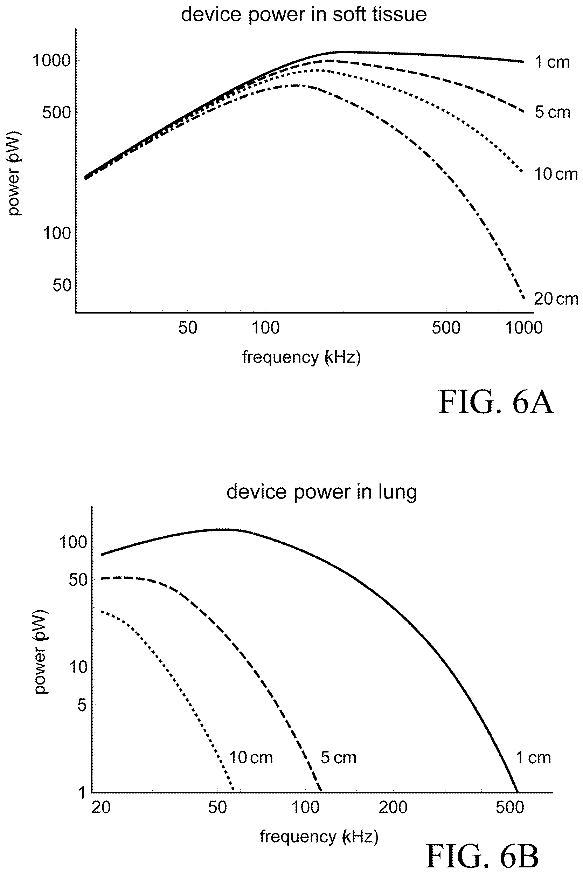

FIGS. 6A and 6B are graphs showing power available to a device 200 when operating at various distances from the skin for a typical low attenuation case (soft tissue), shown in FIG. 6A, and for devices in the lung, shown in FIG. 6B. Note that the frequency and power are shown on log scales. The value next to each curve indicates distance from the skin (for soft tissue) or from the skin-facing surface of the lung (for lung tissue). The graphs indicate that frequencies around 100kHz are well-suited for powering devices in regions with low attenuation between the device and the sound source. Much less power is available for devices operating deep within lung, although using somewhat lower frequencies, e.g., 40kHz, can partially compensate for this attenuation. For devices operating inside biological organisms, frequencies above 50 kHz can be used to avoid uncomfortable audible sound, (Freitas, "Nanomedicine, Volume I: Basic Capabilities," Landes Bioscience, 1999) and for veterinary applications in species with a larger range of hearing, such discomfort may occur at higher frequencies. For example, for many methods, frequencies above about 300 kHz in biological situations suffer from significant attenuation with distance, even without active absorption by devices. For devices operating in other transmissive materials, appropriate frequencies can be determined based on the particular details of the desired operation. For example, for communication, frequencies may be selected based on considerations of both attenuation with distance and optimization of information rate.

These results indicate how other designs could provide more power. For instance, a device could have more pistons, though that would reduce the volume and surface area available for other components. Somewhat larger devices could accommodate more or larger pistons. This could be useful for devices implanted at a fixed location. However, increasing device size is generally not suitable for devices intended to move through circulatory system, which should typically not be much larger than the 2-micron diameter considered here (Freitas, "Nanomedicine, Volume I: Basic Capabilities," Landes Bioscience, 1999). Some examples of larger devices and configurations are analyzed hereafter.

Devices absorbing power decrease the intensity of the pressure wave. It was described above how much power a device absorbs with each piston, i.e., $P_{total}$, of Eq. (11), both for friction and the load. In addition, devices alter the pressure wave due to differences between their acoustic properties and those of the surrounding tissue. In the particular, microscopic devices are likely to be stiffer than biological tissues (Freitas, "Nanomedicine, Volume I: Basic Capabilities," Landes Bioscience, 1999). This gives the devices significantly different acoustic properties than tissue, which lead to scattering and dissipation in boundary layers around the device. The following discussion evaluates these losses, which occur whether or not the device absorbs power.

The sound wavelengths considered here are much larger than the device size (see Table 3). This simplifies the evaluation of scattering and dissipation around the device. In particular, the long wavelength means the sound is insensitive to the distribution of features on the device surface. Moreover, the acoustic pressure at a given time is nearly the same over the entire device. So all cylinders move in phase with each other. Thus, instead of modeling each piston individually, a device's effect on the sound can be estimated by treating the piston motion induced by acoustic pressure variation as its average value, spread uniformly over the device's surface.

Specifically, Eq. (7) gives each piston's motion in response to acoustic pressure $p(t)$. The constant force spring cancels the ambient pressure, so $dx/dt = -p(f)A/k_{total}$. Due to the device's stiffness, the rest of the surface has negligible motion in response to the pressure. With pistons covering a fraction $f_{surface}$ of the surface, the corresponding uniform response is the average of these two cases, i.e., treating the entire surface as having radial velocity $\beta p(t)$ where $\beta = f_{surface}A/k_{total}$. For the device described in Table 5, $f_{surface} = 11\%$. With the parameters of Table 4, full range of piston motion corresponds to about 2% change in sphere radius from this average motion. Thus, a reasonable approximation is that the sphere has a fixed size, i.e., radius $r_{device}$.

Sound moves material slightly back and forth along its direction of propagation. This includes moving the device as a whole. But parts of the device move less relative to each other than the surrounding tissue or fluid due to the higher stiffness of the device.

This approximation of uniform surface motion and a plane wave impinging on the sphere gives an axisymmetric sound field which simplifies the evaluation of scattering and dissipation in the fluid near the sphere. Thus, in the current analysis the sound around a sphere is evaluated in response to an incident plane wave, with boundary condition on the sphere of radial velocity equal to the sum of $-\beta p(t)$ and the radial component of the periodic fluid velocity induced by the plane wave. For long wavelengths, the scattered sound is much weaker than the incident wave (Hilgenfeldt and Lohse, "Response of bubbles to diagnostic ultrasound: a unifying theoretical approach," European Physic Journal, 1998). Thus, a further simplification is to replace $p(t)$ in the boundary condition by the pressure of the incident plane wave. With this simplification, the boundary condition does not depend on the value of the scattered pressure, and evaluating the scattered sound follows the same procedure as for scattering from rigid objects and gas bubbles (Fetter and Walecka, "Theoretical Mechanics of Particles and Continua," Dover Publications, 2003), (Hilgenfeldt and Lohse, "Response of bubbles to diagnostic ultrasound: a unifying theoretical approach," European Physic Journal, 1998), (Sullivan-Silva, "Underwater acoustic scattering from spherical particulates and bubbles," Newport, RI, United States Navy, 1989), but with the boundary conditions for a moveable surface described here.

A useful measure of an object's effect on waves is its attenuation cross section: the ratio of scattered or absorbed power, over a cycle of the wave, to the time-average flux of the incident pressure wave, given by Eq. (13). This attenuation cross section can differ substantially from the geometric surface or cross section areas of the object.

The simplifications described above allow evaluating the scattered sound analytically for wavelengths large compared to the size of the scattering object (Fetter and Walecka, "Theoretical Mechanics of Particles and Continua," Dover Publications, 2003), (Sullivan-Silva, "Underwater acoustic scattering from spherical particulates and bubbles," Newport, RI, United States Navy, 1989). The sound scattered by a sphere is evaluated in response to an incident plane wave using the boundary conditions described above: the radial velocity of the sphere's surface equals the sum of $-\beta p_{inc}(t)$ and the radial component of the periodic fluid velocity induced by the plane wave on the sphere's center of mass. For evaluating scattering, dissipative effects are neglected; these effects are treated separately hereafter.

For this discussion, pressure and velocity are expressed in terms of complex-valued amplitudes $p$ and $v$, respectively, and time-dependence $e^{-i\omega t}$ where $\omega = 2\pi f$ is the angular frequency. The actual values are the real parts, e.g., the pressure is $\Re(pe^{-i\omega t})$, where $\Re$ denotes the real part. For pressure waves, pressure and velocity amplitudes are related by:

$$v = -\frac{i}{\omega\rho}\nabla p \qquad (14)$$

and the amplitudes satisfy the Helmholtz equation:

$$\nabla^2 p + k^2 p = 0 \qquad (15)$$

where $k = \omega/c$ is the wave number. The time-average flux is:

$$\mathcal{F} = \frac{1}{2}\Re(vp^*) \qquad (16)$$

where $p^*$ is the complex-conjugate of $p$ (Fetter and Walecka, "Theoretical Mechanics of Particles and Continua," Dover Publications, 2003).

A convenient representation for matching boundary conditions on a sphere is expressing the pressure in spherical coordinates centered on the sphere with the $z$ direction chosen to be the direction of the incident plane wave's motion. The total sound pressure is the sum of incident and scattered waves: $p = p_{\text{inc}} + p_{\text{s}}$.

The pressure amplitude of the incident plane wave propagating along the $z$-axis is $p_{\text{inc}} = p_0 e^{ikz}$ where $p_0$ is the pressure magnitude of the wave. In spherical coordinates, $z = r\cos(\theta)$, so expressing the plane wave in solutions to the wave equation in spherical coordinates gives the pressure as a sum over modes (Fetter and Walecka, "Theoretical Mechanics of Particles and Continua," Dover Publications, 2003), (Sullivan-Silva, "Underwater acoustic scattering from spherical particulates and bubbles," Newport, RI, United States Navy, 1989):

$$p_{\text{inc}} = p_0 \sum_{m=0}^{\infty} F_m P_m(cos\theta) j_m(kr) \qquad (17)$$

where $F_m = i^m(2m + 1)$, $P_m$ is the Legendre polynomial of order $m$, and $j_m$ is the spherical Bessel function of order $m$. From Eq. (14), the corresponding velocity amplitude, in the $z$ direction, is $v_{\text{inc}} = p_0/(\rho c)e^{ikz}$. The radial component of this velocity is $v_{\text{inc}}\cos(\theta)$.

Similarly, the amplitude of the scattered pressure, $p_{\text{s}}$, is an outgoing wave with expansion (Fetter and Walecka, "Theoretical Mechanics of Particles and Continua," Dover Publications, 2003):

$$p_{\text{s}} = p_0 \sum_{m=0}^{\infty} A_m P_m(cos\theta) h_m^{(1)}(kr) \qquad (18)$$

where $h_m^{(1)}$ is the spherical Hankel function of the first kind of order $m$, and the coefficients $A_m$ are determined by matching the boundary condition at the surface of the sphere, as described below.

At the surface of the sphere, the radial component of the sound's velocity amplitude matches the specified boundary condition. From Eq. (14), this condition is

$$-\frac{i}{\omega\rho}\frac{\partial p}{\partial r} = -\beta p_{\text{inc}} = v_{\text{inc}}\cos(\theta) \qquad (19)$$

evaluated at $r = r_{\text{device}}$, the radius of the sphere and $v_{\text{inc}}$ evaluated at the sphere's center of mass, i.e., $z = 0$. Substituting the above expansions for the incident and scattered waves in this equation gives a relation that must hold for all polar angles $\theta$. This requires matching each mode separately, thereby determining the coefficients $A_m$ of the scattered wave. In particular, since $P_1(x) = x$, the incident velocity $v_{\text{inc}}$ only contributes to mode $m = 1$.

The time-average flux of the incident plane wave, $F_{\text{inc}}$, from Eq. (16) equals Eq. (13) with $p$ replaced by $p_0$. Eq. (16) gives the scattered flux, $\mathcal{F}_{\text{s}}$, in terms of the coefficients $A_m$. For a spherical surface $S$ of radius $r$ centered on the scattering sphere, the total scattered power is the integral of $\mathcal{F}_{\text{s}}$ over the surface $S$. Thus the scattering cross section is

$$\sigma = \frac{1}{\mathcal{F}_{\text{inc}}}\int_S \mathcal{F}_s \, dS \qquad (20)$$

where $dS = r^2 \sin(\theta)d\theta d\varphi$ is the differential surface area for the sphere, with polar and azimuthal angles, $\theta$ and $\varphi$, respectively. The scattered sound spreads over the sphere but is not attenuated in this model. Thus the integral is independent of radius r.

When the sound wavelength is large compared to the sphere's radius, i.e., $kr_{device}$ 1, only modes $m = 0$ and $m = 1$ contribute significantly to the scattered sound. This long-wavelength limit applies to the scenarios considered here (see Table 2). In this case, the coefficients are

$$A_0 = \frac{2}{3}\beta c\rho(kr_{device})^4 - \frac{1}{3}i(kr_{device})^3 - \beta c\rho(kr_{device})^2$$

$$A_1 = -\frac{1}{2}i\beta c\rho(kr_{device})^4 \qquad (21)$$

Using these values in Eq. (20) gives the scattering cross section

$$\pi r_{device}^2 \left( 4(kr_{device})^2(\beta c\rho)^2 + \frac{4}{9}(kr_{device})^4(1 - 12(\beta c\rho)^2) \right) \quad (22)$$

The first factor, $\pi r_{device}^2$ , is the geometric cross section of the device. Since $kr_{device} \ll 1$, the scattering cross section is much smaller than the device's size.

A hard sphere corresponds to $\beta = 0$, for which Eq. (22) becomes (4/9) $\pi r_{device}^2(kr_{device})^4$ (Hilgenfeldt and Lohse, "Response of bubbles to diagnostic ultrasound: a unifying theoretical approach," European Physic Journal, 1998). This is Rayleigh scattering, proportional to the fourth power of the sound's frequency (Rayleigh, "The principle of similitude," Nature, 1915). If the sphere's center of mass is held in place rather than able to move in response to the wave, the cross section is a bit larger: the fraction is 7/9 instead of 4/9.

Near the device surface, viscous and thermal effects alter the sound propagation, which leads to dissipation. This occurs in viscous and thermal boundary layers with characteristic lengths $\sqrt{2\eta/(\rho\omega)}$ and $\sqrt{2k_{thermal}/(\rho C_p\omega)}$ for viscous and thermal dissipation, respectively (Fetter and Walecka, "Theoretical Mechanics of Particles and Continua," Dover Publications, 2003). For the scenarios considered here, these boundary layers extend about a micron from the device surface, a distance comparable to the size of the device.

These effects are evaluated here using viscous and thermal properties of water or blood plasma, given in Table 2. This is reasonable for devices in the bloodstream: because the boundary layers are small, the properties of blood determine behavior in the boundary layers around the devices, rather than the properties of the tissues through which the vessels are passing. This contrasts with the use of tissue attenuation properties for the sound propagation (see Table 1), since the wavelengths are large compared to size of most vessels.

The dissipation is evaluated numerically using the approximations described above. As expected for sound in liquids, viscous dissipation is much larger than that due to thermal effects. Dividing this dissipation by the flux of the plane wave gives the cross section for dissipation in the fluid around the sphere.

Viscous losses in the boundary layer around the sphere arise from no-slip condition at the surface of the sphere. Appropriately designed surfaces could reduce this dissipation, as discussed above. In addition to nanoscale surface structure to reduce viscous drag, the device surface may be designed to allow some tangential motion, e.g., because combinations of both radial and tangential motion can improve the efficiency of locomotion based on surface vibrations (Hogg, "Using surface-motions for locomotion of microscopic robots in viscous fluids," J. of Micro-Bio Robotics, 2014). A surface with some tangential motion, along with the radial piston motion, could more closely match the acoustic motion of fluid when there is no device. Such a surface will have less effect on the sound wave than a rigid surface, and hence less viscous dissipation.

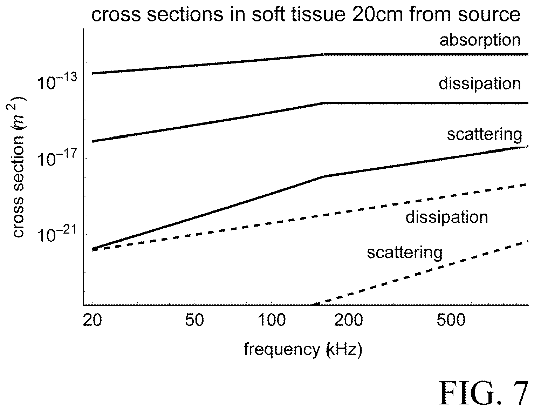

FIG. 7 shows the contributions of processes removing energy from a plane wave for a device in soft tissue 20cm from the ultrasound source, the same scenario as illustrated by the bottom curve in FIG. 6A. The cross sections vs. frequency is plotted for a single device, with 20 pistons, located in soft tissue 20cm from a 50kPa transducer. The curves show cross sections for energy absorbed by the pistons, dissipation in the fluid around the device, and energy scattered by the device. For comparison, the dashed curves show cross sections for dissipation and scattering by a hard sphere (corresponding to a device with pistons locked at their limit of motion so they do not respond to the acoustic pressure changes). The dotted horizontal line is the geometric cross section of the device, $\pi r_{\text{device}}^2$. The vertical dotted line is the frequency, 160kHz, at which 2 = 2 (see Eq. (8)). The total cross section for removing energy from the sound wave is the sum of these three contributions. As illustrated in FIG. 7, absorption is the dominant contribution to the cross section for the cases considered here. That is, the moving pistons extract significantly more energy from the sound wave than dissipation in the viscous boundary layer around the device, which in turn is a much larger effect than the scattered energy. FIG. 7 also shows that a hard sphere scatters and dissipates sound in a boundary layer, but this attenuation is several orders of magnitude less than that from devices absorbing power.

Applications using a large number of devices may cause significant attenuation, significantly reducing signal power with increasing distance from the transmission source. In a typically application, this can reduce the power available to devices located further from the transmission source. This is a particular concern for scenarios where the power source is outside the body: devices near the path from the skin to devices deep within the body could significantly attenuate sound reaching deep into the body. The following discussion evaluates attenuation due to many devices, using the effect of a single device on the sound as described above, to quantify these effects with the example scenarios given in Table 6. These examples use various numbers of devices in a body with volume $V_{\text{body}}$ = 50L. The typical spacing is the average distance between neighboring devices, estimated as the cube root of the average volume per device.

Table 6: Scenarios for multiple devices in a body with 50L volume

| Number of devices | Typical spacing | Number density |
|---|---|---|
| $10^{10}$ | $170\mu$m | $2 \times 10^{11}$/m³ |
| $10^{11}$ | $80\mu$m | $2 \times 10^{12}$/m³ |
| $10^{12}$ | $40\mu$m | $2 \times 10^{13}$/m³ |

The analysis assumes incoherent scattering from different devices. In this case, attenuation is the product of the number density of devices and the attenuation cross section of a single device. For devices with number density $v_{\text{device}}$ and cross section $\sigma$, amplitude attenuation is

$$\alpha_{\text{device}} = \frac{1}{2} v_{\text{device}} \sigma \qquad (23)$$

The attenuation for power is twice this value.

For estimating the effect of multiple devices, the devices are considered to be uniformly distributed throughout the body, so $v_{\text{device}}$ is the ratio of number of devices to the body volume. In practice, there could be some variation in this value. E.g., if devices are uniformly distributed in the blood volume, then parts of the body with high or low blood supply will have correspondingly higher or lower $v_{\text{device}}$. If, instead, devices concentrate in a portion of the body, e.g., a single organ, the corresponding number density for a given number of devices will be larger in that region, with correspondingly larger attenuation and smaller elsewhere. The number density of devices employed is dependent on the particular operation being performed. For example, when micron-sized devices are employed, the methods herein are discussed in terms of a number density of devices ranging from $10^{10}$ to $10^{12}$ for a 50L volume. For larger devices, the number density can be reduced proportionally to the increase in volume per device to retain a similar total volume of the devices. In these examples, devices ranging in size from 1 micron radius to 1mm radius are addressed, although devices outside these ranges could be employed in particular operations.

As noted above, FIG. 7 shows that energy absorbed by the pistons is the dominant contribution to the cross section. Thus, a calculation of power absorption is used to evaluate the effect of large numbers of devices.

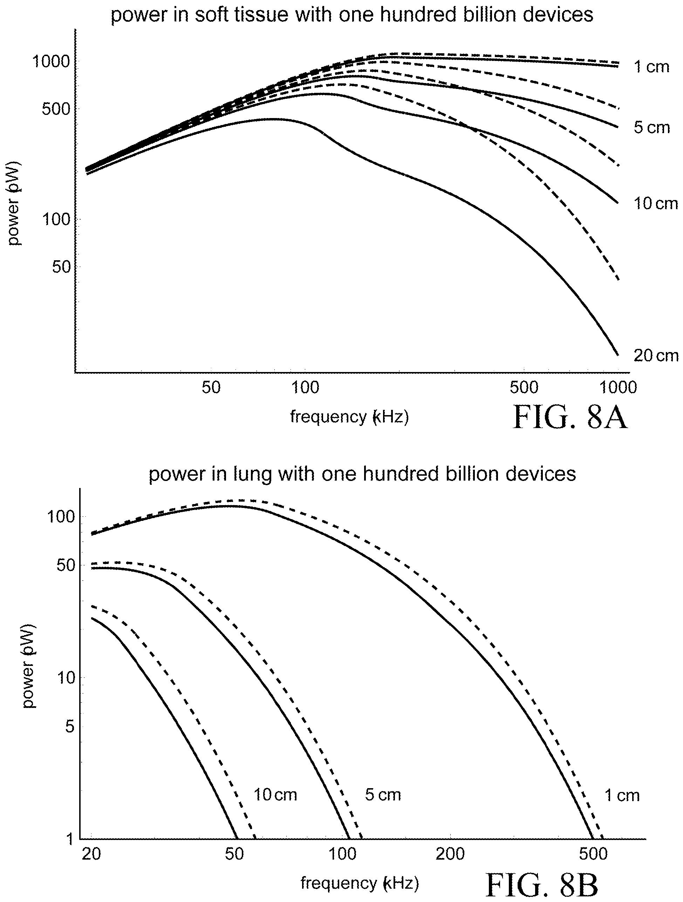

FIGS. 8A and 8B are graphs of power vs. frequency at different depths where multiple devices are actively absorbing the acoustic signal. These graphs shows the effect of $10^{11}$ devices on power at various distances from the source, for soft tissue (8A) and for lung tissue (8B). Power with attenuation due to devices collecting power (solid curves) are plotted for the scenario of Table 5, and dashed curves show power vs. frequency without device attenuation, from FIGS. 6A and 6B.

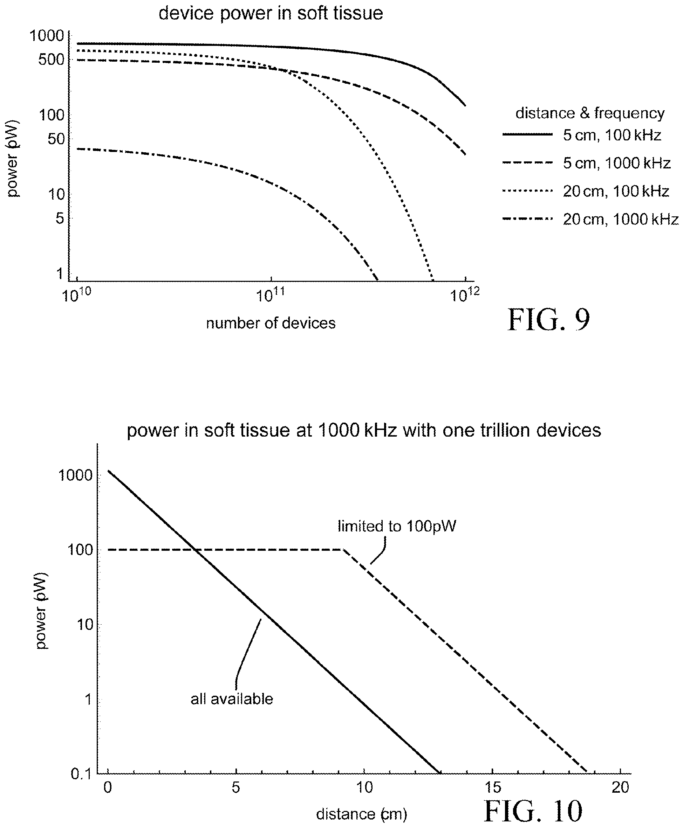

Comparing with FIGS. 6A & 6B shows that this number of devices significantly reduces power in soft tissue at the higher frequencies and distances. On the other hand, attenuation in lung is so large that the additional attenuation due to devices is relatively minor. Attenuation in soft tissue increases rapidly with additional devices, as shown in FIG. 9, which plots power, with attenuation due to devices collecting power in soft tissue, as a function of number of devices uniformly distributed in the body volume for the scenario of Table 5. The curves correspond to the indicated distances from the source and the sound frequencies.

As discussed above, large numbers of devices extracting power significantly increases attenuation in tissue that would otherwise provide significant power to deeper devices. The following discussion describes some methods of operating devices that could be used to mitigate this problem. Combinations and variations of these methods could be employed, and in some cases, devices may be operated differently at different times or locations, to flexibly allocate power in spite of the additional attenuation due to power extraction.

In one method of operation, devices can adjust their activities based on available power. For example, devices have more power when they are near the skin than when deep in tissue. Thus, a device could defer power-intensive tasks, such as data analysis or communication, until they are near the skin. Conversely, devices deeper in tissue could perform a more limited set of tasks, or only operate intermittently.

Instead of using the power as received, a device could store energy for later use (Freitas, "Nanomedicine, Volume I: Basic Capabilities," Landes Bioscience, 1999). Stored energy can provide higher burst power, or power when the device is in a location where the sound is too attenuated to provide adequate power. Energy storage allows separating when energy is collected from when it is used to drive loads in the device.

Energy storage could also be useful when using a mix of device sizes. That is, larger devices, stationed at fixed locations rather than being small enough to travel through capillaries, could collect and store energy. By vibrating their surfaces, such devices could act as small transducers distributed within the tissue, providing power to smaller devices as they pass nearby. This power transmission would occur over much smaller distances than power from external transducers, so could use higher frequencies that would significantly attenuate if used by the external transducers. These larger devices could both provide power and communication, with the larger efficiency provided by the higher frequencies (Hogg and Freitas, "Acoustic communication for medical nanorobots," Nano Communication Networks, 2012).

Devices near the skin could reduce their contribution to attenuation by reducing their power collection. One way they could do so is by reducing the number of active pistons by locking some in place. Alternatively, a device could reduce the power collected by each piston. This could involve slower motion (by increasing the load on each piston) or stopping pistons during a portion of each acoustic cycle (e.g., by reducing the range of piston motion). For the same power, the latter approach leads to more dissipation, and hence higher attenuation.

Stopping piston motion over several acoustic cycles rather than just part of one has an additional possible benefit if devices near the skin synchronize their duty cycles for absorbing power: while all devices near the skin stop absorbing power, they reduce sound attenuation, making bursts of higher power available to deeper tissue. This contrasts with unsynchronized duty cycles, which only somewhat increases average power to deeper devices. A synchronization signal could be added to the sound wave from transducers, or could be provided from clocks in the devices.

Another option is to select the range of pistons so high-pressure variation pushes them to their limits. While stuck at their limits, pistons do not absorb power and can be similar in stiffness to the rest of the device surface. In this case, devices less than half a wavelength apart would automatically be approximately synchronized by experiencing extreme pressure variations at about the same time. This is particularly useful for devices intended to operate near the skin. Such devices can get significant power without a large range for pistons and will have more of their volume available for other uses.

Collecting less power reduces the major contribution to acoustic attenuation even though these devices still attenuate sound through dissipation in the boundary layer and by scattering (see FIG. 7). As one example of using this method of operation, FIG. 10 shows a situation with such a large number of devices ($10^{12}$ devices) that only a little power of a 1000KHz signal would remain 20cm from the source (see FIG. 9) if no mitigation method were employed. In the method shown in FIG. 10, devices that limit their power considerably increase the depth at which other devices can receive substantial power. The curves in FIG. 10 compare the power with distance when devices use all available power and when they are each limited so as to use no more than 100pW.

Using two or more frequencies can provide more power to devices deeper in the body. In one example of this approach, devices near the skin extract power from a higher frequency, while a lower frequency, with lower attenuation, is reserved for deeper devices. By not extracting power from the low frequency, the devices closer to the transmission source only passively attenuate the sound, i.e., by scattering and viscous dissipation in fluid around the device. This passive attenuation is significantly less than attenuation due to power extraction (see FIG. 7). Devices could select to absorb high or low frequency with a prespecified distance, i.e., built into the robot control by the designer; assuming that the devices can sense their distance or receive commands from another device that can. Alternative criteria can be used to determine frequency selection; for example, devices could be adaptive, briefly trying each frequency, then picking the one giving the most power (such as where the shorter-range, higher frequency is transmitted at greater strength than the lower, longer range frequency); as another example, devices could use a ratio of power from high and low frequencies to decide when to switch (e.g., using the values indicated in FIG. 11 or FIGS. 16-18 discussed below). Devices could switch among these methods as they move, or as the available power changes, e.g., due to movement of other robots closer to the source. One limitation of using split frequencies for medical or veterinary applications is that the combined intensity of the frequencies cannot exceed the safe limit on total intensity. Thus, splitting the sound among two or more frequencies means less power from each frequency than if using that frequency alone.

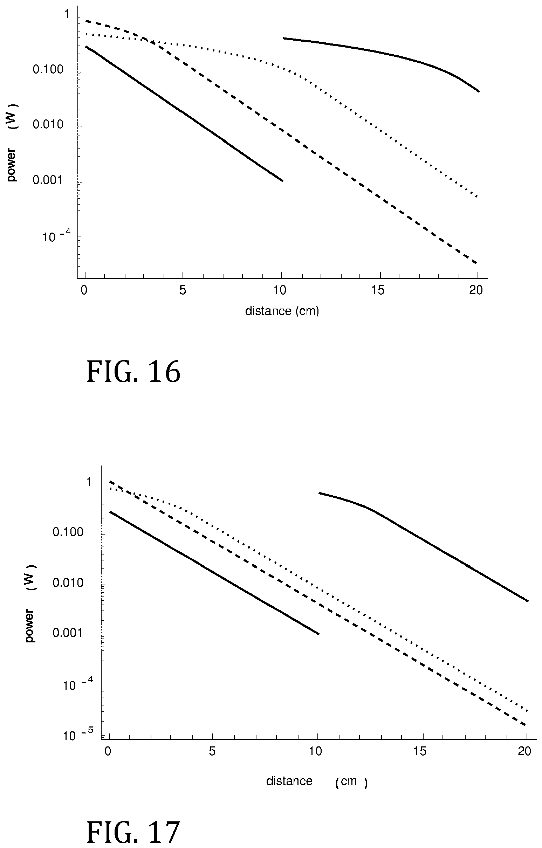

FIG. 11 shows an example of this method of operation. In this method, devices monitor the power available from the higher frequency. They use that frequency for power when it provides at least 2.3pW, which occurs at a distance of 8cm from the source. Otherwise, they switch to the lower frequency (note that device "distance" here is determined by signal strength, rather than distance *per se*). FIG. 11 shows this method used for $10^{12}$ devices. The solid curves show power for the scenario of Table 5, except the $1000W/m^2$ source intensity is split between 100kHz and 300kHz, with source pressures 43kPa and 23kPa, respectively. Compared to using just one of these frequencies for all devices, this split-frequency approach provides significantly more power to deeper devices. In this method, devices 10cm from the source have more power than those a bit closer to the skin. Thus, acoustic power need not decrease monotonically with distance into the body. Some additional examples of transmitting on different frequencies are discussed below with regard to FIGS. 16-18.

One way in which this technique can be accomplished is for devices near the skin to adjust their springs to avoid responding to the low frequency, in the same way they compensate for variations in ambient pressure, as discussed with Eq. (7). For example, where the acoustic signal is transmitted on two frequencies (and ignoring any ambient pressure and phase differences), the total pressure as a function of time is the combined pressures from the two frequencies:

$$p_{total}(t) = p_1 \cos(\omega_1 t + \varphi_1) + p_2 \cos(\omega_2 t + \varphi_2) \qquad (24)$$

Where $\varphi_1$ and $\varphi_2$ are the phase angles of the two frequencies. The constant-force spring of each device located near the transmission source can be adjusted at a frequency matching that of the lower frequency acoustic signal to effectively cancel it out, in the same manner as adjusting to compensate for ambient pressure in the case of devices operating in the circulatory system.

As noted above, ambient pressure changes for devices that operate in the circulatory system, creating an effective pressure cycle of about 1 per minute frequency for overall circulation, and about 1 Hz frequency when operating in an artery. The adjustment of the piston response to pressure can be adjusted to compensate for these changes. These changes are much slower than those used to select a frequency of absorption. A possible control feedback for the device is measuring the average position of the piston over the relevant time scale (e.g., tens of seconds for circulation, milliseconds for heart beat). The difference between this measured average and, for example, the midway position of the piston between the limits on its range, could be the control error signal, and the device could adjust the force on the springs to reduce the error, using a conventional control method (e.g., PID controller).

As an example of adjustment to select a frequency of absorption, if frequencies are split between 100 kHz and 300 kHz (as discussed for FIG. 11), closer devices can adjust their constant-force springs to compensate for the 100kHz pressure variation (thus absorbing only the 300 kHz signal). This compensation (performed, for example, under the direction of a controller such as a PID controller) requires changing the overlap, $L_{spring}$, much more rapidly than needed to adjust for changes in ambient pressure. As an extreme case, consider a device close to a source with 50kPa pressure amplitude. It should adjust for pressure changing by twice this amount each half-period of the pressure wave. This corresponds to changing $L_{spring}$ by about 35nm in $5\mu$s. From Eq. (5) and using the upper bound on $L_{spring}h_{spring}$ of $0.01\mu m^2$, this adjustment dissipates less than $10^{-3}$ pW. While far larger than the power dissipated to adjust to ambient pressure variation, this dissipation is much less than the power available to the device. While more distant devices could adjust to absorb only the 100 kHz frequency, there is typically no advantage to doing so, as the 300 kHz frequency is largely attenuated before reaching such devices.

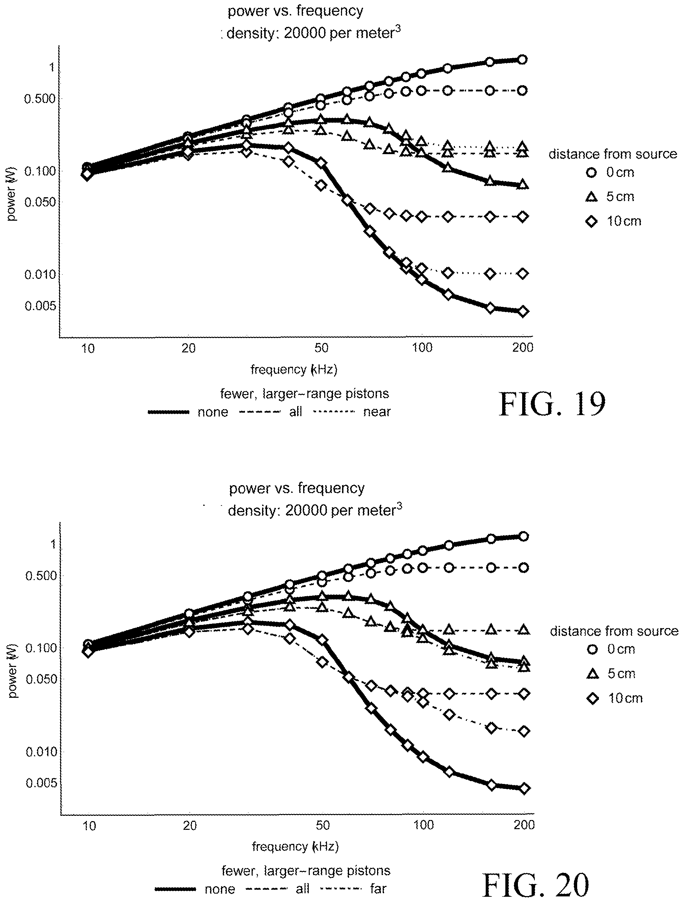

In some applications, devices may remain in one location for an extended period of time. In such cases, problems with attenuation can be reduced by employing different classes of devices. E.g., devices designed to work in high-power environments near the skin could only use high frequencies and hence could have fewer, shallower pistons, since their range of motion is less at high frequencies. Such pistons would not be as effective at collecting power at lower frequencies. On the other hand, devices intended for deeper operation could devote more of their volume to pistons to more efficiently collect power from the lower frequency waves that penetrate deeper. That is, a heterogeneous mixture of device designs could better match the availability of acoustic power than if all devices have the same designs. The use of different types of devices is discussed further with regard to FIGS. 19A-22B.

In situations where it is desired to transmit an acoustic signal to a particular location, devices could avoid certain paths between the transmission source and such location, allowing more sound energy to reach the desired location. As one example, devices close to the skin could avoid positions between the skin and deeper tissue, thereby avoiding attenuation of the signal to devices in those deeper regions. These regions may not be static, e.g., some tissue moves a few centimeters during each breath, which is larger than the wavelengths considered here. Devices should adjust for this motion if it changes the locations of devices relative to the deeper locations that require the additional power. If deeper devices do not require continuous power, devices could only employ this mitigation occasionally, as needed, thereby alternating power between deeper and shallower devices. In this way, devices manage energy collection and use as a group, rather than each device focusing on the energy it needs for its own use. This approach is especially useful if the main power-using activities are in relatively small deep regions.

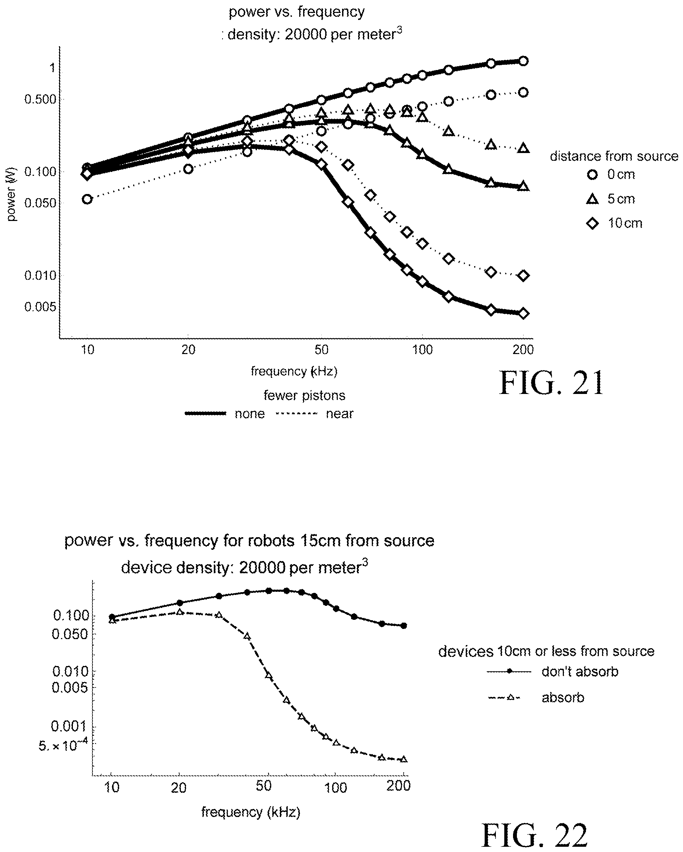

FIG. 12 illustrates the effect of one example of this method of operation, where devices avoid paths between transducers on the skin and the surface of the lung. In this case, the number of devices is large enough ($10^{12}$ devices) to considerably attenuate the sound in the tissue between the lung and the skin (see FIG. 9). Avoiding those locations provides more power to devices in the lung. The solid curves in FIG. 12 show power when devices avoid locations between the lung surface and the skin. For comparison, the dashed curves show power when uniformly distributed devices absorb power at all locations, including between the lung and the skin. The values next to the curves indicate distance from the skin-facing surface of the lung. Further examples of these methods are discussed below with regard to FIGS. 22-23D.

Ideally, for this method of operating the devices, the devices would actively avoid the regions that block the transmission path to the desired location. Thus, they would not contribute to attenuation by any of the mechanisms compared in FIG. 7. However, moving devices out of some areas means higher concentrations in others, thereby increasing attenuation in those areas above the values discussed herein for a uniform distribution of devices.

As an alternative to moving out of the path, devices could achieve much of the same effect by stopping or reducing their power absorption when they detect they are passing through path locations. Such devices would still attenuate sound due to scattering and dissipation near their surfaces, but much less than when they actively absorb power (see FIG. 7).

In another method of operation, devices capable of measuring their distances to neighbors could position themselves to precisely tune acoustic properties at scales well below the wavelength, thereby creating dynamic acoustic metamaterials (Chen and Chan, "Acoustic cloaking and transformation acoustics," Journal of Physics D: Applied Physics, 11, IOP Publishing, 2010), (Zheludev, "The road ahead for metamaterials," Science, 2010) to manipulate the sound field. This could include focusing sound into small, deeper regions where devices require additional power to perform their tasks.

Frequencies around 100kHz provide the most power in much of the body. However, devices more than a centimeter or so within high-attenuation tissue receive little power. Even within a tissue with low attenuation, power can vary with small-scale changes in device location. This is because power decreases with increasing viscosity of the fluid around the device (see Eq. (12)). The scenarios discussed here correspond to devices in blood vessels. Devices outside of blood vessels could encounter much larger viscosities (Freitas, "Nanomedicine, Volume I: Basic Capabilities," Landes Bioscience, 1999), and hence have less power than nearby devices in vessels. In summary, ultrasound is a feasible power source, but the amount of power varies significantly with the device's location in the body at both macro and micro scales.

Acoustic power poses challenges for device design. While pistons are relatively simple energy collectors, they are also bulky, using a significant fraction of device volume and surface, compared, for example, to chemical power from fuel cells (Freitas, "Nanomedicine, Volume I: Basic Capabilities," Landes Bioscience, 1999), (Hogg and Freitas, "Chemical Power for microscopic robots in capillaries," Nanomedicine: Nanotechnology, Biology and Medicine, 2, 2010). Thus, using enough pistons to collect adequate power may reduce performance of uses of the device volume or surface. For example, a device may require substantial volume for tanks of chemicals, energy storage or information processing. The device could require surface area for a variety of tasks. In many cases, one important task is surface devoted to chemical sensors or pumps. Such devices can be much smaller than the total surface area, allowing room for a large number of them (Freitas, "Nanomedicine, Volume I: Basic Capabilities," Landes Bioscience, 1999). For this task, pistons are not likely to be a major issue because chemical collection is only weakly dependent on the fraction of surface area absorbing the chemical (Berg, "Random Walks in Biology," Princeton University Press, 1993). However, area devoted to pistons could be more limiting for other uses. For example, communication (Hogg and Freitas, "Acoustic communication for medical nanorobots," Nano Communication Networks, 2012) or locomotion (Hogg, "Using surface-motions for locomotion of microscopic robots in viscous fluids," J. of Micro-Bio Robotics, 2014) can require substantial surface area. A possible mitigation to these competing uses is for the pistons to perform multiple functions. For example, the actuators adjusting the spring's force in response to changing ambient pressure could also provide force to move the pistons. This would readily provide surface oscillations for locomotion, which involve lower frequencies (1-10kHz) but similar range of motion (Hogg, "Using surface-motions for locomotion of microscopic robots in viscous fluids," J. of Micro-Bio Robotics, 2014) as the pistons considered here. On the other hand, actuating the pistons for acoustic communication requires much higher frequencies and smaller ranges of motion (Hogg and Freitas, "Acoustic communication for medical nanorobots," Nano Communication Networks, 2012), which may be beyond the capabilities of actuators adjusting the springs in response to ambient pressure changes.

The discussion above is primarily focused on micron-sized devices, as such devices have particular interest for medical applications due to their ability to move readily through the circulatory system. However, the operating methods discussed are applicable for any situation where a large number of devices are operated within a transmissive material For examples of such applications, the following analysis considers millimeter-size devices (1000x larger than the devices addressed in the above discussion), where the devices are assumed to be operating in a large tank of water, with acoustic power delivered from the walls of the tank. Plane waves entering the tank from part or all of its surface. The power available to the devices as a function of distance to the source (i.e., closest transmitter on the surface, assuming the distance is small enough that plane waves are a good approximation) is evaluated. To simplify calculations, it is assumed that other surfaces of the tank are sufficiently far enough away or are absorbing so there is no need to account for reflections and interference from those surfaces. These assumptions parallel the setting as used above for evaluating micron-size devices operating in tissue.

Compared to the above analysis of micron-size devices in tissue, this example differs in several ways. It should be kept in mind that these are offered as two examples to illustrate the effectiveness of the present operating methods to mitigate attenuation, and should not be seen as limiting; these methods should be beneficial for operating multiple devices at a wide range of sizes and in different situations.

Water, as used for this example, has a lower viscosity compared to biological fluids, and this lower viscosity and larger device size means the low Reynolds number approximation is not as accurate. Lower acoustic attenuation in the fluid (water) compared to biological tissue for the distances and number of devices evaluated means that substantially all the attenuation is due to devices, rather than also having substantial contribution from tissue (e.g., lungs). The larger device size means the devices are closer in size to the wavelengths studied here than for micron-size devices, which reduces the accuracy of the analysis of passive scattering by devices when they are not absorbing power, i.e., scattering from hard spheres, since that analysis assumes devices are much smaller than the wavelength (see Table 3 above). For such larger devices, it may be beneficial to employ corresponding lower frequencies (for example, frequencies having a wavelength at least 10x larger than the device size), which would no longer be ultrasound, and could have wavelengths comparable to or larger than the size of the tank. Devices in a tank of water exposed to atmospheric pressure have constant pressure (i.e., no time variation), unlike the pressure variation in blood vessels, especially arteries, due to the heartbeat. Thus, the larger devices in this example do not need to adjust to changing ambient pressure (which is also true of micro-devices when operating in situations where surrounding pressure is constant). They do, however, have variation in hydrostatic pressure based on their depth in the water. This is analogous to the location-based pressure variation for micron-size devices moving from arteries to veins. Piston friction in these larger devices is dominated by viscous drag, as with the analysis for atomically precise micron-size devices, and it is assumed that viscous damping is much larger than sliding friction of the piston as it moves in its housing. This is reasonable for atomically smooth surfaces, based on the estimate of sliding friction coefficient, $k_{sliding} = 10^3$ kg/(m$^2$ s). This value presumably greatly underestimates sliding friction for MEMS devices. If sliding friction is actually large enough to equal or surpass drag due to viscosity, each device will extract less power and will not attenuate the sound as much. This is not an important difference for illustrating mitigation methods: if devices attenuate less than assumed in this discussion, then the results shown here would correspond to the actual attenuation from a corresponding larger number of devices.

Table 7 lists the acoustic properties and device parameters used in the following discussion. The amplitude absorption coefficient is frequency dependent (NMI Table 4.2, p. 117) For the scattering cross section, the above discussion of Eq. 14 notes when devices lock pistons, they scatter and dissipate as hard spheres, giving the attenuation due to devices not absorbing power. The number density of devices is selected to provide the same total volume as in the micro-device examples. The devices here are 1000x larger than the micron-scale devices, hence each having 10$^9$ times larger volume. 10$^{12}$ micron-size devices in 50L body volume have a number density $2 \times 10^{13}$ devices/m$^3$; the corresponding number for millimeter-size devices would be 10$^9$ times smaller, i.e., $2 \times 10^4$ devices/m$^3$. With this number density, the typical spacing of uniformly distributed devices is about 37 mm. For the acoustic intensity, there is no need to restrict to the biological safety limit as in the micro-device example; however, the same value of 1000 W/m$^2$ is employed

$$\mathcal{F} = \frac{1}{2} \frac{p^2}{\rho c}$$

here for definiteness. Time-average flux is        ; for intensity of 1000 W/m$^2$, this corresponds to an acoustic pressure variation of 55 kPa, and allowing for some losses at the water surface, a convenient choice for the incoming pressure is $p$ = 50 kPa. The viscous drag coefficient for cylinder motion, $k_{viscous}$ is defined in terms of drag force when it moves at speed v through the fluid: $F_{drag} = k_{viscous}$ v with $k_{viscous} = g\eta d_{piston}$ where $\eta$ is fluid viscosity and $d_{piston}$ the diameter of the piston. This example uses the same viscous drag factor, $g$ = 45, as used for the micron-size analysis; thus, increasing piston diameter by a factor of 1000 increases $k_{viscous}$ by the same factor.

Table 7: Parameters for millimeter-size devices example

| Acoustic properties | | |
|---|---|---|
| Speed of sound | $c$ | 1500 m/s |
| Fluid density | $\rho$ | 1000 kg/m$^3$ |

(continued)

| Acoustic properties | | |
|---|---|---|
| amplitude absorption coefficient | $\alpha_{coef}$ | $2.5 \times 10^{-14}$ s$^2$/m |
| Source pressure | $\rho$ | 50 kPa |
| Device parameters | | |
| radius | $r$ | 1 mm |
| Number of pistons | $n_{pistons}$ | 20 |
| Piston range of motion | $h_{piston}$ | 200 $\mu$m |
| Piston cross-section area | Apiston | 0.071 mm$^2$ |
| Duty cycle for piston operation | | 50% |
| Viscous drag coefficient for piston | $k_{viscous}$ | $1.35 \times 10^{-5}$ sN/m |
| Device number density | $v$ | $2 \times 10^4$/m$^3$ |
| Typical device spacing | | 37 mm |

The wavelength large compared to device size, e.g., 100kHz is 15mm, 20kHz is 75mm, 1kHz is 1500mm (from Table 3).

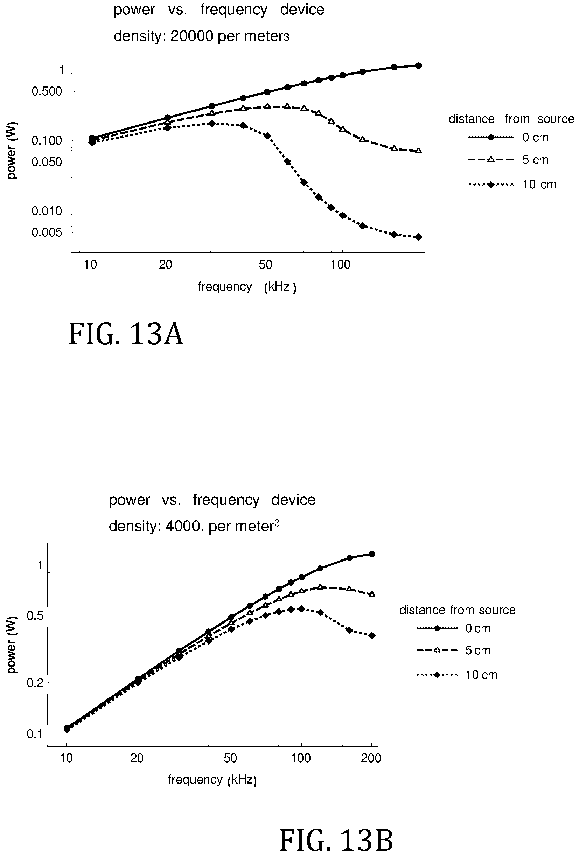

As a basis for comparison, FIGS. 13A & 13B illustrate available power as a function of frequency for devices at three distances from the transmission source, for larger (1mm dia.) devices operating in a body of water. Note that the axes are logarithmically scaled. The number density of devices for FIG. 13A (20,000/meter$^3$) is selected to have the same total volume as for the 1-micron devices illustrated in FIGS. 8A & 8B, while the number density of devices for FIG. 13B is much smaller (4,000/meter$^3$). The attenuated pressure is evaluated by numerically solving the attenuation differential equation

$$dp(x)/dx == -\alpha(x) * p(x) \qquad (25)$$

where $\alpha(x)$ is the attenuation coefficient, with contributions from attenuation due to the fluid (which is small in these examples) and due to devices absorbing power, which depends on the pressure, hence making this a nonlinear equation. It can be seen that, for higher frequencies, available power is attenuated significantly with increased distance from the transmission source.

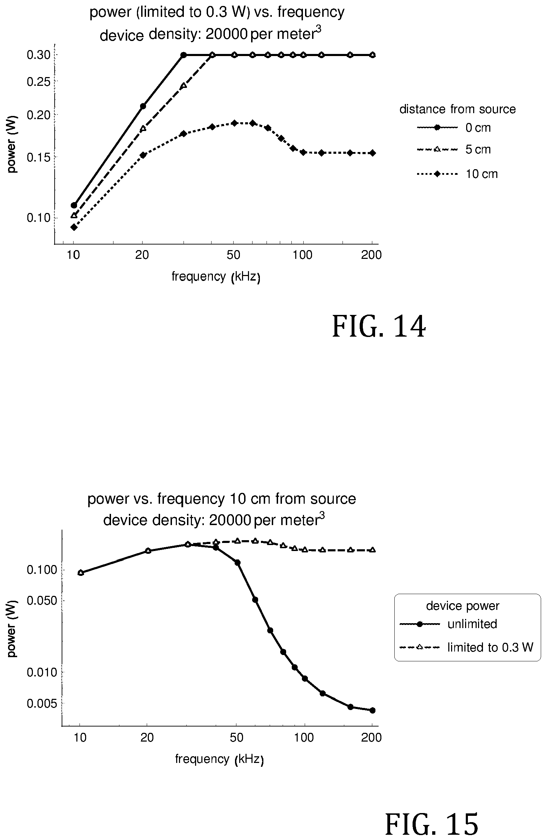

FIGS 14 and 15 illustrate the effect of having devices limit the amount of power that they absorb, rather than each device absorbing as much power as is available to it at its location. Such limiting could be done based on location (for example, all devices close to the transmission source limit their power absorption) and could be done in a coordinated manner (for example, all devices near the transmission source could cease collecting power together for periods, to allow a burst of relatively unattenuated signal to a location further from the source).

FIG. 14 illustrates the available power vs. frequency at three distances, in a manner similar to the situation shown in FIG. 13A; however, in this case the power absorption by each device is capped at 0.3W. To show the effect of this method at greater distances, FIG. 15 compares the power vs. frequency curves for available power at 10cm distance from the source, these curves being taken from FIG. 13A and FIG. 14 but presented with a different vertical scale. The difference for higher frequencies (within the range analyzed herein) is clearly visible. When devices are allowed to absorb without the 0.3W limit, devices near the source significantly attenuate the sound, leaving little signal power available for deeper devices at high frequencies.

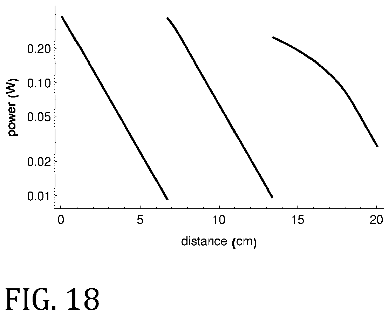

As discussed above with regard to FIG. 11 (for the example of micron-sized devices operating in biological tissue), a signal can be transmitted on more than one frequency, and devices can absorb different frequencies depending on their relative distance from the transmission source. FIGS. 16A-18 illustrate the effect of such an operating method in some examples of frequency splits. These examples compare available power with increasing distance from the transmission source for pairs of frequencies (FIGS. 16 & 17), and a generalization to an example where three frequencies are employed (for near, middle and deep devices) (FIG. 18). The relation between average flux and pressure does not depend on frequency, so these examples use the same division of source pressures for low and high frequencies as was used in the example shown in FIG. 11 for 100kHz and 300kHz, i.e., 43kPa for the lower frequency and 25kPa for the higher frequency.

In any of these cases, the response of the devices can be adjusted for absorption of particular frequencies in a manner similar to that discussed above for micron-sized devices. For example, when pistons are employed to absorb energy from the signal, the spring stiffness of the piston can be adjusted (by use of adjustment means such as employed for high-precision watches, by MEMS actuators, etc.) and/or the piston can be directly forced (such as by using MEMS actuators or similar means), with the frequency and magnitude of adjustment selected to effectively cancel out the undesired frequency or frequencies. As discussed above with regard to FIG. 11, various criteria can be used to determine when a device selects a particular frequency for absorption. Examples are selecting frequency based on location (such as distance from the transmission source(s) or location within a specified path), based on received signal strength, etc. Devices may also change frequencies in coordination with other devices, for example periodically switching frequencies to reduce or increase absorption of a particular frequency during such periods.

In the first method, shown in FIG. 16, the signal is split between 50kHz and 100KHz, with devices within 10 cm of the transmission source absorbing only at 100kHz, and those further absorbing at 50 kHz. The solid lines in FIG. 16 show the power with distance when this method is employed, while the broken lines shows the power with distance for the case where the entire signal is transmitted at 50KHz (dotted line) and the case when transmitted at 100KHz (dashed line).

FIG. 17 illustrates a similar method, but where the signal is split between the frequencies of 100KHz and 300KHZ. Again, the solid lines show the power with distance when devices within 10 cm of the transmission source absorb only at 300kHz and those further absorb at 100KHZ, while the broken lines show the power with distance for the cases where all the signal is transmitted at 100KHz (dotted line) and at 300KHZ (dashed line).

FIG. 18 illustrates the available power with distance for a situation where the signal is split across three frequencies (50kHz, 100kHz, and 300kHz), with the transmission power split equally among the three, so each frequency has 29kPa, (i.e., 50 kPa / $\sqrt{3}$), and each frequency used for 1/3 of the 20cm distance (thus, devices from 0-6.7cm absorb at 300kHz, devices from 6.7-13.4cm absorb at 100kHz, and devices from 13.4-20cm absorb at 50kHz. Where multiple frequencies are employed, devices within a particular distance range may adjust only to avoid absorbing the frequencies used by those devices located more distant. For the example shown in FIG. 18, the devices located from 0-6.7 cm adjust to avoid absorbing both the 50 kHz and 100 kHz frequencies, and devices located from 6.7-13.4 cm adjust to avoid absorbing the 50 kHz frequency.

Another method, when devices are intended to operate at known locations, is to employ different devices (or differently-operated devices) at different distances from the transmission source. The devices differ in their signal-absorbing characteristic. For purposes of illustration, the examples discussed here differ in number and/or configuration of pistons employed to absorb energy from the signal; however, other characteristics of the devices could be varied. FIGS. 19-21 illustrate some examples of such methods. In the example shown in FIGS. 19 & 20, absorption as a function of frequency is compared for devices having many, small-range pistons vs. devices having fewer, large-range pistons - in both cases, using the same total volume of pistons (this is appropriate if all devices need a fixed fraction of their volume for other components, e.g., computer, binding sites, etc. ) FIG. 21 compares the cases where the number of pistons is varied, but the size of the pistons remains constant.

For FIGS. 19 & 20, devices as discussed above in Table 7 are considered in comparison to devices having half as many pistons, but where each piston has twice the range. These devices with fewer, larger-range pistons absorb less power near the transmission source, so they attenuate less and allow more power to be received by deeper devices.

FIG. 19 compares three methods, two of which make use of the limited absorption of devices having fewer pistons when operated close to the transmission source. One method shown is the same as for FIG. 13, where only devices having more, smaller-range pistons are employed (solid lines, with the data point shapes indicating the distance from the signal source). In another method (dashed lines), all devices have half the number of pistons, but each pistons has twice the operating range. In the third method, devices having fewer, larger-range pistons are operated within 3cm of the transmission source, and devices having the original piston number and size (as set forth in Table 7) are operated at more than 3cm from the source. FIG. 19 illustrates the resulting power vs. frequency at three distances. Note that, at the 0 cm distance, the curves for the two methods where fewer piston devices are used overlap. Comparing these methods, it can be seen that the use of mixed device types (dotted lines) results in more available energy at 5cm and 10cm depths for higher frequencies compared to using all devices with more pistons. For the method using all fewer-piston devices (dashed lines), the results are mixed, with less available power than the mixed device method at some depths and frequencies, but more power at the 10cm depth for higher frequencies. This suggests that the mixture of devices and frequency of operation can be optimized for a particular intended application. Such optimization may also depend on the use of the signal. The above discussion focuses on signal strength, which may be the primary consideration for situations where the signal is used to transmit power (such as to provide power to devices, to apply acoustic energy to a target location for therapeutic reasons, etc.) When the signal is intended for communication, another consideration may be optimization of information rate. Such optimization typically depends on the frequency bandwidth as well as on the signal to noise ratio, according to the Shannon-Hartley theorem

$$C = B \log_2 \left( 1 + \frac{S}{N} \right) \qquad\qquad (26)$$

where C is the channel capacity, $B$ is the bandwidth, and $S/N$ is the signal to noise ratio. The signal to noise ratio is dependent on signal strength as well as on noise, and in situations where the transmissive material is subject to noise at particular frequencies, the frequencies selected for transmission may be made to avoid such frequencies where significant noise is present. Where devices communicating (or exterior sources communicating with devices) are a significant source of noise, it may be advantageous for devices to adjust their frequencies of transmission and/or absorption to reduce the noise at particular locations where reception would otherwise be difficult.

FIG. 20 illustrates the reverse situation to that shown in FIG. 19, where devices with fewer, larger-range pistons are operated more than 3cm from the signal source, and devices within 3cm of the source have the original piston number and size from Table 7. FIG. 20 compares this method (dotted lines) to the methods where all devices have more pistons (solid lines) and where all devices have fewer pistons (dashed lines). For the 0 cm distance, the curve for the mixed-device method overlaps the curve for the method where all devices have more pistons. In this method (dotted lines), comparison with the method where all devices have more pistons (solid lines) shows that the use of mixed device types results in more available energy at the 10cm depth for higher frequencies, but somewhat less at this depth and at 5cm depth for lower frequencies. Comparing to the method where all devices have fewer pistons (dashed lines), the use of mixed device types results in less power for 5cm and 10cm depths at higher frequencies, and no benefit for these depths at lower frequencies. While there is more power available at 0cm depth for the mixed devices, this is of little practical benefit since providing sufficient power to devices close to the transmission source is seldom a concern.

FIG. 21 compares the method where all devices have more pistons to an alternative situation employing mixed device types. Here, the devices differ in the volume of their available internal space. Thus, devices located further than 3 cm of the signal source have the parameters set forth in Table 7, while those devices that are within 3 cm have half the number of pistons, but where the pistons are the same size (as contrast with the situation discussed for FIG. 19, where the pistons of devices having fewer pistons have twice the range of movement). This example simulates a situation where some devices devote much of their volume to pistons, giving high power but relatively little space for other components; while other devices have less piston volume, and more volume for other components. If the low-volume devices mainly operate near the source, they could get sufficient power with fewer pistons. In terms of power, this situation is equivalent to a method in which those devices having a full set of pistons operate them with a smaller duty cycle, or where they cease operation of half their pistons (however, in such cases, the pistons would still occupy volume, so the devices would not have more space for other components).

FIG. 21 plots the available power as a function of frequency at three depths, comparing the situation (solid lines) where all devices have the original piston number and size (as set forth in Table 7) to the situation (dotted lines) where devices with fewer pistons are located within 3cm of source, and devices have the original piston number are located at greater distances. The comparison shows that the use of fewer pistons for closer devices results in more power at the 5 cm and 10cm depth for higher frequencies. The devices with fewer pistons have less power at 0 cm distance, but this is generally not a concern, as the available power close to the transmission source is seldom a limiting factor. While the devices with fewer pistons absorb less power (thereby leaving more available for deeper devices), the devices with fewer pistons at closer distances still have more power than deeper ones. This is similar to shallow devices limiting their power absorption (as discussed above with regard to FIG. 10), but in this case, the shallow devices would have more internal volume free for other components. This illustrates one trade-off between these options: devices with fewer pistons can carry more of other components, but they need to operate closer to the source to get power. Devices that limit power have the flexibility to operate at either shallow or deep locations, since they can use more pistons when they are deeper, to somewhat compensate for the attenuated acoustic pressure. At low frequencies, devices with fewer pistons get less power than deeper devices with the larger number of pistons.

In situations where information is available on the locations of devices (typically determined internally by the device monitoring input and/or determined by an external device and either the location information or operating instructions being communicated to the device from the external device), the devices can be operated to avoid actively absorbing the acoustic signal when within a path between the transmission source and a location further from the transmission source than the device is. Two general methods for achieving this result are for devices within such path and near the source to avoid actively absorbing the signal (so as to only create passive attenuation), and for devices having locomotion capability to move away from or avoid such paths (so no attenuation results from these devices near the transmission source). Since the fluid itself has very little attenuation over centimeter distances, the second method is similar in effect to reducing the distance of deep devices to the surface. E.g., if devices within 5cm of the surface move away, then devices at 10cm depth have the same power 5cm-deep devices would have when devices are uniformly distributed. In this case, fluid and scattering when devices don't absorb power is very small, so these two methods are similar. In effect, devices not absorbing or clearing a path within distance d of the source gives deeper devices the same power they would have gotten if they were distance d closer to the source when all devices are absorbing power. This situation is similar to that discussed above with regard to FIG. 12 for micron-sized devices operating within lung tissue.

FIG. 22 illustrates one example of such a method, comparing the available power vs. frequency at 15cm depth for the method where devices within 10cm cease actively absorbing (dashed line) and when they do not (solid line). The 15 cm distance curve for the method when devices at shallower depths are not actively absorbing is very similar to the curve for the 5cm depth in FIG. 13A, showing that when devices within 10cm of the source do not absorb, the effect at depth 15cm is essentially the same as for devices at 5cm depth in the method where all devices are actively absorbing the signal.

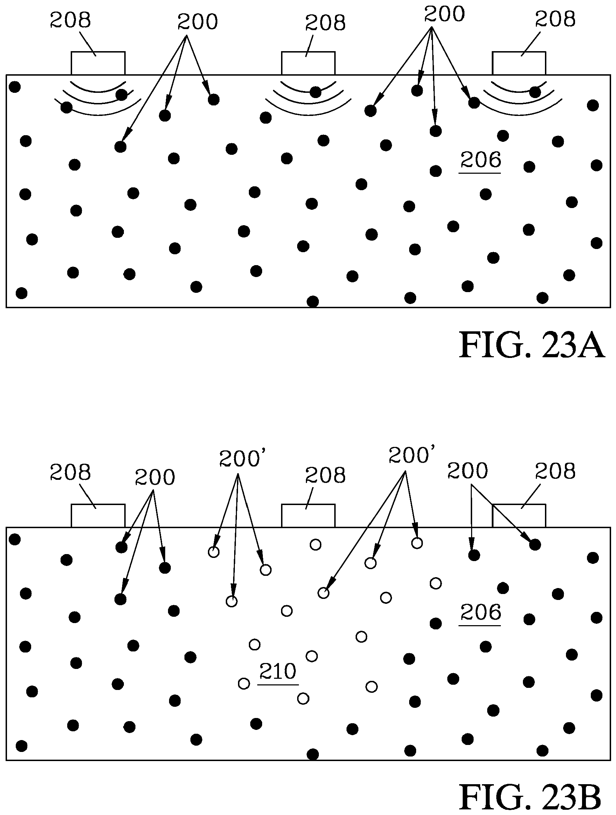

FIGS. 23A-23D illustrate some examples of operating devices 200 such as shown in FIG. 5 distributed throughout a transmissive material 206. The devices 200 are operated to reduce attenuation of a signal transmitted by transducers 208 along a path 210 to a desired location, providing a situation similar to those shown in the power vs. frequency graph of FIG. 22 (and similar to the situation shown in FIG. 12, except that in that case there is a difference in material with distance from the transmission source). In the method shown in FIGS. 23A & 23B, a control signal is generated by the transducers 208 (in addition to the primary signal being absorbed), and received by the devices 200. Those devices 200' which are located within the path 210 cease (or significantly reduce) actively absorbing the primary signal, allowing the signal to be received by devices 200 that are located further from the transducers 208. While shown clearing a path to further devices, such a path could be employed when the signal is being transmitted to some other desired target area.

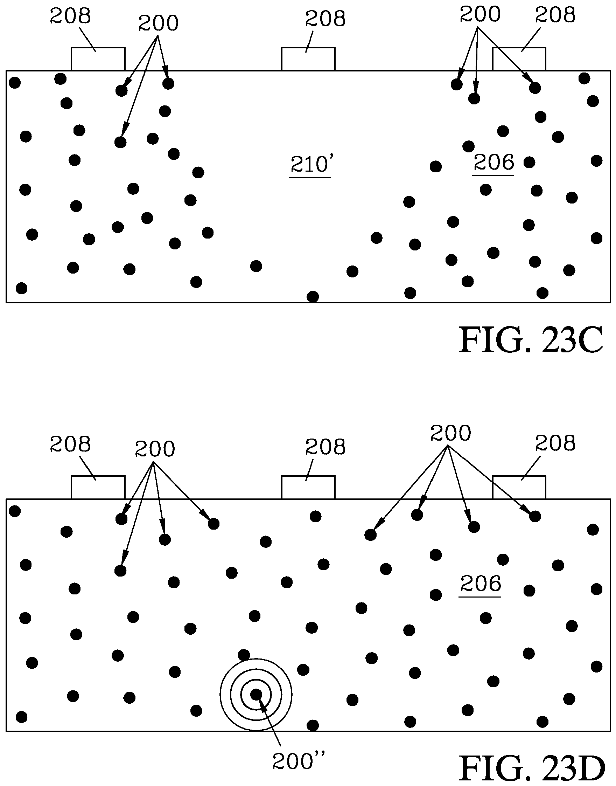

FIG. 23C illustrates an alternative method to that shown in FIG. 23B, where devices 200 having locomotion capability have moved out of the path 210 in response to the control signal, rather than ceasing operation. Note that while this reduces absorption of the signal in the path 210, it increases absorption in the surrounding material 206, due to the increased number of devices 200 actively absorbing the signal there.

FIG. 23D illustrates another alternative method. The situation shown is similar to that shown in FIG. 23A, but here the control signal is transmitted by the device 200" that is located distant from the transducers 208. In response to this signal, those devices that are between the device 200" and the nearest transducer 208 can either cease actively absorbing (as shown in FIG. 23B) or can move out of the path (as shown in FIG. 23C). This method can allow adequate power to the device 200" when the device 200" needs a relatively large amount of power for a short period, such as to recharge an energy storage device, transmit a burst of data, or similar temporary high-energy activity.

In any of the methods, a control signal can be transmitted when the avoidance of absorption in the path 210 is no longer needed, and the devices 200 can either resume actively absorbing (for the method shown in FIG. 23B) or move back into the path 210 (for the method shown in FIG. 23C).

It should be appreciated that the various mitigation methods discussed above may be used in combination when such is beneficial for a particular situation. For example, the method of limiting signal absorption could be used where the discussion addresses ceasing signal absorption, and methods discussed for reducing or ceasing signal absorption under certain circumstances could be conducted based on additional considerations, such as based on location, timing, coordination by communicated signal, etc. And, these methods relating to operation could be combined with the use of mixed device types and/or operation of different devices on different frequencies. Additionally, where methods and device operating parameters are discussed in terms of location, such location could be determined based on absolute location (such as location within a specified organ in a body) or relative location (such as distance or position relative to the transmission source, other devices, interfaces between different organs, etc.), according to the needs of a particular situation and desired operating purpose.

Bibliography

Berg, H., (1993), "Random Walks in Biology," Princeton University Press.

Chen, H. and Chan, C. T. (2010), "Acoustic cloaking and transformation acoustics", Journal of Physics D: Applied Physics.

Cumings, J. and Zettl, A. (2000), "Low-friction nanoscale linear bearing realized from multiwall carbon nanotubes", Science.

Drexler, K. E., (1992), "Nanosystems: Molecular Machinery, Manufacturing, and Computation," New York, John Wiley & Sons.

Fetter, A. L. and Walecka, J. D., (2003), "Theoretical Mechanics of Particles and Continua," Dover Publications.

Freitas, R., (1999), "Nanomedicine, Volume I: Basic Capabilities," Landes Bioscience.

Happel, J. and Brenner, H., (1983), "Low Reynolds Number Hydrodynamics," The Hague, Kluwer.

Hilgenfeldt, S. and Lohse, D. (1998), "Response of bubbles to diagnostic ultrasound: a unifying theoretical approach", European Physic Journal.

Hogg, T. (2014), "Using surface-motions for locomotion of microscopic robots in viscous fluids", J. of Micro-Bio Robotics.

Hogg, T. (2018), "Stress-Based Navigation for Microscopic Robots in Viscous Fluids", Journal of Micro-Bio Robots.

Hogg, T. and Freitas, R. (2010), "Chemical Power for microscopic robots in capillaries", Nanomedicine: Nanotechnology, Biology and Medicine.

Hogg, T. and Freitas, R. (2012), "Acoustic communication for medical nanorobots", Nano Communication Networks.

Hogg, T., Moses, M. S., et al., (2017), "Evaluating the Friction of Rotary Joints in Molecular Machines."

Liu, Z., Liu, J. Z., et al. (2012), "Interlayer binding energy of graphite: A mesoscopic determination from deformation", Physical Review B.

Mate, C. M., (2008), "Tribology on the Small Scale: A Bottom Up Approach to Friction, Lubrication, and Wear," OUP Oxford.

Purcell, E. M. (1977), "Life at low Reynolds number", American J. of Physics.

Rayleigh, L. (1915), "The principle of similitude", Nature.

Sullivan-Silva, K. B., (1989), "Underwater acoustic scattering from spherical particulates and bubbles," Newport, RI, United States Navy.

Zheludev, N. L. (2010), "The road ahead for meta materials", Science.

**Claims**

1. A method of operating a plurality of devices (200), the devices (200) being configured to extract energy from a mechanical wave signal, the method comprising the steps of:

   distributing the devices (200) throughout a transmissive material (206);
   transmitting a mechanical wave signal into the transmissive material (206) from at least one transmission source (208); **characterized in**
   adjusting the operation of devices (200) based on their proximity to the transmission source (208) to limit the attenuation of the mechanical wave signal, which is caused by the extraction of the mechanical wave energy by devices (200) closer to the transmission source (208), as received by at least one selected location further from the transmission source (208).

2. The method of claim 1, wherein said step of adjusting the operation of the devices (200) comprises:
   reducing the absorption of the signal by closer devices (200) when such closer devices (200) are operating, compared to the absorption of the signal by further devices (200), by having at least a subset of the closer devices (200) deactivate at least a subset of signal-absorbing structures.

3. The method of claim 1, wherein said step of adjusting the operation of the devices (200) comprises:
   reducing the absorption of the signal by closer devices (200) when such closer devices (200) are operating, compared to the absorption of the signal by further devices (200), by having at least a subset of the closer devices (200) adjust the operating characteristics of at least one signal-absorbing structure to reduce the resulting signal absorption.

4. The method of claim 1, wherein said step of adjusting the operation of the devices (200) comprises reducing the time of active absorption of the signal by at least a subset of the closer devices (200).

5. The method of claim 4, wherein at least a subset of the closer devices (200) cease actively absorbing the signal during coordinated periods.

6. The method of claim 1, wherein said step of adjusting the operation of the devices (200) comprises reducing the absorption of the signal by a subset of the devices (200), based on their locations.

7. The method of claim 6, wherein at least a subset on the devices (200) use locomotion to avoid, while they are actively absorbing the signal, locations where they impede transmission of the signal to at least one selected location further from the transmission source (208).

8. The method of claim 6, wherein at least a subset of the closer devices (200) reduce their absorption of the signal when in locations where they impede transmission of the signal to at least one selected location further from the transmission source.

9. The method of claim 6, wherein a subset of the devices (200) that are designed to have limited absorption of the signal are operated in locations closer to the transmission source (208) than another subset of devices (200) that are not as limited in their absorption.

10. The method of claim 1, wherein the signal is transmitted on more than one frequency, and wherein said step of adjusting the operation of the devices (200) comprises operating at least a subset of the devices (200) such that the frequency at which they primarily absorb the signal is determined by their position relative to the transmission source (208).

11. The method of claim 10, wherein at least a subset of the devices (200) closer to the transmission source (208) primarily absorb the signal at a higher frequency than devices (200) further from the transmission source (208).

12. The method of one of claims 1 to 11, wherein the signal is a pressure wave signal.

13. The method of one of claims 1 to 12, wherein the step of distributing the devices (200) throughout a transmissive material (206) further comprises distributing at least 20,000 devices per $M^3$.

**Patentansprüche**

1. Verfahren zum Betreiben einer Vielzahl von Vorrichtungen (200), wobei die Vorrichtungen (200) so ausgestaltet sind, dass sie einem Signal einer mechanischen Welle Energie entziehen, wobei das Verfahren die folgenden Schritte umfasst:

   Verteilen der Vorrichtungen (200) in einem gesamten durchlässigen Material (206);
   Übertragen eines Signals einer mechanischen Welle in das durchlässige Material (206) hinein ausgehend von mindestens einer Übertragungsquelle (208); **gekennzeichnet durch**
   Anpassen des Betriebs der Vorrichtungen (200) basierend auf ihrer Nähe zu der Übertragungsquelle (208), um die Dämpfung des Signals der mechanischen Welle einzuschränken, welche durch die Entziehung der Energie der mechanischen Welle seitens Vorrichtungen (200), die sich näher zu der Übertragungsquelle (208) befinden, verursacht wird, wenn dieses von mindestens einem ausgewählten Ort, der sich weiter weg von der Übertragungsquelle (208) befindet, empfangen wird.

2. Verfahren nach Anspruch 1, wobei der Schritt des Anpassens des Betriebs der Vorrichtungen (200) Folgendes umfasst:
   Verringern der Absorption des Signals seitens näher befindlicher Vorrichtungen (200) während des Betriebs dieser näher befindlichen Vorrichtungen (200), verglichen mit der Absorption des Signals seitens weiter weg befindlicher Vorrichtungen (200), indem zumindest eine Teilmenge der näher befindlichen Vorrichtungen (200) dazu gebracht wird, zumindest eine Teilmenge von signalabsorbierenden Strukturen zu deaktivieren.

3. Verfahren nach Anspruch 1, wobei der Schritt des Anpassens des Betriebs der Vorrichtungen (200) Folgendes umfasst:
   Verringern der Absorption des Signals seitens näher befindlicher Vorrichtungen (200) während des Betriebs dieser näher befindlichen Vorrichtungen (200), verglichen mit der Absorption des Signals seitens weiter weg befindlicher Vorrichtungen (200), indem zumindest eine Teilmenge der näher befindlichen Vorrichtungen (200) dazu gebracht wird, die Betriebseigenschaften von zumindest einer signalabsorbierenden Struktur anzupassen, um die resultierende Absorption des Signals zu verringern.

4. Verfahren nach Anspruch 1, wobei der Schritt des Anpassens des Betriebs der Vorrichtungen (200) ein Verringern der Zeitdauer einer aktiven Absorption des Signals seitens zumindest einer Teilmenge der näher befindlichen Vorrichtungen (200) umfasst.

5. Verfahren nach Anspruch 4, wobei zumindest eine Teilmenge der näher befindlichen Vorrichtungen (200) ein aktives Absorbieren des Signals während koordinierter Zeiträume einstellt.

6. Verfahren nach Anspruch 1, wobei der Schritt des Anpassens des Betriebs der Vorrichtungen (200) ein Verringern der Absorption des Signals seitens einer Teilmenge der Vorrichtungen (200) basierend auf ihren Orten umfasst.

7. Verfahren nach Anspruch 6, wobei zumindest eine Teilmenge der Vorrichtungen (200) Ortsveränderung verwendet, um, während sie das Signal aktiv absorbieren, Orte zu vermeiden, an denen sie eine Übertragung des Signals zu mindestens einem ausgewählten Ort, der sich weiter weg von der Übertragungsquelle (208) befindet, behindern.

8. Verfahren nach Anspruch 6, wobei zumindest eine Teilmenge der näher befindlichen Vorrichtungen (200) ihre Absorption des Signals verringert, wenn sich diese an Orten befinden, an denen sie eine Übertragung des Signals zu mindestens einem ausgewählten Ort, der sich weiter weg von der Übertragungsquelle befindet, behindern.

9. Verfahren nach Anspruch 6, wobei eine Teilmenge der Vorrichtungen (200), die so ausgebildet sind, dass sie eine eingeschränkte Absorption des Signals aufweisen, an Orten betrieben wird, die sich näher zu der Übertragungsquelle (208) befinden, als eine andere Teilmenge von Vorrichtungen (200), die hinsichtlich ihrer Absorption nicht so eingeschränkt sind.

10. Verfahren nach Anspruch 1, wobei das Signal auf mehr als einer Frequenz übertragen wird, und wobei der Schritt des Anpassens des Betriebs der Vorrichtungen (200) ein Betreiben zumindest einer Teilmenge der Vorrichtungen (200) derart umfasst, dass die Frequenz, auf der sie das Signal hauptsächlich absorbieren, durch ihre Position in Bezug auf die Übertragungsquelle (208) bestimmt wird.

11. Verfahren nach Anspruch 10, wobei zumindest eine Teilmenge der Vorrichtungen (200), die sich näher zu der Übertragungsquelle (208) befinden, das Signal mit einer höheren Frequenz absorbiert als Vorrichtungen (200), die sich weiter weg von der Übertragungsquelle (208) befinden.

12. Verfahren nach einem der Ansprüche 1 bis 11, wobei das Signal ein Druckwellensignal ist.

13. Verfahren nach einem der Ansprüche 1 bis 12, wobei der Schritt des Verteilens der Vorrichtungen (200) in einem gesamten durchlässigen Material (206) ferner ein Verteilen von mindestens 20.000 Vorrichtungen pro m$^3$ umfasst.

**Revendications**

1. Procédé de fonctionnement d'une pluralité de dispositifs (200), les dispositifs (200) étant configurés pour extraire de l'énergie à partir d'un signal d'onde mécanique, le procédé comprenant les étapes suivantes :

   répartition des dispositifs (300) à travers un matériau transmissif (206) ;
   transmission d'un signal d'onde mécanique dans le matériau transmissif (206) à partir d'au moins une source de transmission (208) ; **caractérisé par**
   le réglage du fonctionnement des dispositifs (200) sur la base de leur proximité avec la source de transmission (208) pour limiter l'atténuation du signal d'onde mécanique, laquelle est causée par l'extraction de l'énergie d'onde mécanique par des dispositifs (200) plus proches de la source de transmission (208), tel que reçu par au moins un emplacement sélectionné plus éloigné de la source de transmission (208).

2. Procédé selon la revendication 1, dans lequel ladite étape de réglage du fonctionnement des dispositifs (200) comprend :
   la réduction de l'absorption du signal par des dispositifs (200) plus proches lorsque ces dispositifs (200) plus proches fonctionnent, en comparaison avec l'absorption du signal par des dispositifs (200) plus éloignés, où au moins un sous-ensemble des dispositifs (200) plus proches désactive au moins un sous-ensemble des structures d'absorption de signal.

3. Procédé selon la revendication 1, dans lequel ladite étape de réglage du fonctionnement des dispositifs (200) comprend :
   la réduction de l'absorption du signal par des dispositifs (200) plus proches lorsque de tels dispositifs (200) plus proches fonctionnent, en comparaison avec l'absorption du signal par des dispositifs (200) plus éloignés, où au moins un sous-ensemble des dispositifs (200) plus proches règle les caractéristiques de fonctionnement d'au moins une structure d'absorption de signal pour réduite l'absorption de signal résultante.

4. Procédé selon la revendication 1, dans lequel ladite étape de réglage du fonctionnement des dispositifs (200) comprend la réduction du temps d'absorption active du signal par au moins un sous-ensemble des dispositifs (200) plus proches.

**5.** Procédé selon la revendication 4, dans lequel au moins un sous-ensemble des dispositifs (200) plus proches cesse d'absorber activement le signal pendant des périodes coordonnées.

**6.** Procédé selon la revendication 1, dans lequel ladite étape de réglage du fonctionnement des dispositifs (200) comprend la réduction de l'absorption du signal par un sous-ensemble des dispositifs (200), sur la base de leurs emplacements.

**7.** Procédé selon la revendication 6, dans lequel au moins un sous-ensemble des dispositifs (200) utilise la locomotion pour éviter, pendant que ceux-ci absorbent activement le signal, des emplacements où ils empêchent la transmission du signal vers au moins un emplacement sélectionné plus éloigné de la source de transmission (208).

**8.** Procédé selon la revendication 6, dans lequel au moins un sous-ensemble des dispositifs (200) plus proches réduit leur absorption du signal lorsque ceux-ci se trouvent à des emplacements où ils empêchent la transmission du signal vers au moins un emplacement sélectionné plus éloigné de la source de transmission.

**9.** Procédé selon la revendication 6, dans lequel un sous-ensemble des dispositifs (200) conçus pour avoir une absorption limitée du signal fonctionne à des emplacements plus proches de la source de transmission (208) qu'un autre sous-ensemble de dispositifs (200) moins limités dans leur absorption.

**10.** Procédé selon la revendication 1, dans lequel le signal est transmis sur plus d'une fréquence, et dans lequel ladite étape de réglage du fonctionnement des dispositifs (200) comprend le fonctionnement d'au moins un sous-ensemble des dispositifs (200) de telle façon que la fréquence à laquelle ceux-ci absorbent prioritairement le signal est déterminée par leur position relative par rapport à la source de transmission (208).

**11.** Procédé selon la revendication 10, dans lequel au moins un sous-ensemble des dispositifs (200) plus proche de la source de transmission (208) absorbe prioritairement le signal à une fréquence plus élevée que des dispositifs (200) plus éloignés de la source de transmission (208).

**12.** Procédé selon l'une des revendications 1 à 11, dans lequel le signal est un signal d'onde de pression.

**13.** Procédé selon l'une des revendications 1 à 12, dans lequel l'étape de répartition des dispositifs (200) à travers un matériau transmissif (206) comprend en outre la répartition d'au moins 20.000 dispositifs par $M^3$.

FIG. 1

FIG. 2

FIG. 3

FIG. 4

FIG. 5A

FIG. 5B

FIG. 5C

FIG. 5D

FIG. 5E

device power in soft tissue

FIG. 6A

device power in lung

FIG. 6B

cross sections in soft tissue 20cm from source

FIG. 7

power in soft tissue with one hundred billion devices

FIG. 8A

power in lung with one hundred billion devices

FIG. 8B

device power in soft tissue

distance & frequency

——— 5 cm, 100 kHz

--- - 5 cm, 1000 kHz

········· 20 cm, 100 kHz

–·–·–·· 20 cm, 1000 kHz

FIG. 9

power in soft tissue at 1000 kHz with one trillion devices

limited to 100pW

all available

FIG. 10

power in soft tissue with one trillion devices

FIG. 11

power in lung with one trillion devices

FIG. 12

power vs. frequency device
density: 20000 per meter3

FIG. 13A

power vs. frequency device
density: 4000. per meter$^3$

FIG. 13B

FIG. 14

FIG. 15

FIG. 16

FIG. 17

FIG. 18

power vs. frequency
density: 20000 per meter³

distance from source
O 0 cm
△ 5 cm
◇ 10 cm

frequency (kHz)

fewer, larger-range pistons
——— none  – – – all  ········ near

FIG. 19

power vs. frequency
density: 20000 per meter³

distance from source
O 0 cm
△ 5 cm
◇ 10 cm

frequency (kHz)

fewer, larger-range pistons
——— none  – – – all  ······· far

FIG. 20

FIG. 21

FIG. 22

FIG. 23A

FIG. 23B

FIG. 23C

FIG. 23D

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8743659 B
- US 8743660 B
- US 8755252 B
- US 8760972 B
- US 8787115 B
- US 8837258 B
- US 10024950 B
- WO 2015187742 A2
- US 10024950 B1

**Non-patent literature cited in the description**

- Wireless Optogenetic Nanonetworks: Device Model and Charging Protocols. **STEFANUS A. WIRDATM-ADJA et al.** ARXIV.ORG. Cornell University Library, 201 Olin Library Cornell University, 20 June 2017, 14853
- Long-Term Alleviation of Parkinsonian Resting Tremor Using Wireless Optogenetic Nanonetworks. **MISRA SUDIP et al.** IEEE TRANSACTIONS ON NANOBIOSCIENCE. IEEE SERVICE CENTER, 12 March 2020, vol. 19, 403-409
- A Sub-mm'3 Ultrasonic Freefloating Implant for Multi-mote Neural Recording. **MOHAMMAD MERAJ GHANBARI et al.** ARXIV.ORG. CORNELL UNIVERSITY LIBRARY, 201 OLIN LIBRARY CORNELL UNIVERSITY, 19 May 2019, 14853
- **FREITAS.** Nanomedicine, Volume I: Basic Capabilities. *Landes Bioscience,* 1999
- **HOGG ; FREITAS.** Acoustic communication for medical nanorobots. *Nano Communication Networks,* 2012
- **HOGG.** Using surface-motions for locomotion of microscopic robots in viscous fluids. *J. of Micro-Bio Robotics,* 2014
- **HOGG.** Stress-Based Navigation for Microscopic Robots in Viscous Fluids. *Journal of Micro-Bio Robots,* 2018
- **CUMINGS ; ZETTL.** Low-friction nanoscale linear bearing realized from multiwall carbon nanotubes. *Science,* 2000
- Interlayer binding energy of graphite: A mesoscopic determination from deformation. **LIU, LIU et al.** Physical Review B. American Physical Society, 2012, vol. 20
- **HAPPEL ; BRENNER.** Low Reynolds Number Hydrodynamics. The Hague, Kluwer, 1983
- **BERG.** Random Walks in Biology. Princeton University Press, 1993
- **MATE.** Tribology on the Small Scale: A Bottom Up Approach to Friction, Lubrication, and Wear. OUP Oxford, 2008
- **DREXLER.** Nanosystems: Molecular Machinery, Manufacturing, and Computation. John Wiley & Sons, 1992
- **FREITAS.** Nanomedicine, Volume I: Basic Capabilities. Landes Bioscience, 1999
- **HOGG, MOSES et al.** Evaluating the Friction of Rotary Joints in Molecular Machines. *arXiv.org,* 2017
- **PURCELL.** 45. *American J. of Physics,* 1977
- **HILGENFELDT ; LOHSE.** Response of bubbles to diagnostic ultrasound: a unifying theoretical approach. *European Physic Journal,* 1998
- **FETTER ; WALECKA.** Theoretical Mechanics of Particles and Continua. Dover Publications, 2003
- **HILGENFELDT AND LOHSE.** Response of bubbles to diagnostic ultrasound: a unifying theoretical approach. *European Physic Journal,* 1998
- **SULLIVAN-SILVA.** Underwater acoustic scattering from spherical particulates and bubbles. Newport, RI, 1989
- **RAYLEIGH.** The principle of similitude. *Nature,* 1915
- Basic Capabilities. **FREITAS.** Nanomedicine. Landes Bioscience, 1999, vol. I
- Acoustic cloaking and transformation acoustics. **CHEN ; CHAN.** Journal of Physics D: Applied Physics. IOP Publishing, 2010, vol. 11
- **ZHELUDEV.** The road ahead for metamaterials. *Science,* 2010
- **HOGG ; FREITAS.** Chemical Power for microscopic robots in capillaries. *Nanomedicine: Nanotechnology, Biology and Medicine,* 2010, vol. 2
- **BERG, H.** Random Walks in Biology. Princeton University Press, 1993
- **CHEN, H ; CHAN, C. T.** Acoustic cloaking and transformation acoustics. *Journal of Physics D: Applied Physics,* 2010

- **CUMINGS, J ; ZETTL, A.** Low-friction nanoscale linear bearing realized from multiwall carbon nanotubes. *Science,* 2000
- **DREXLER, K. E.** Nanosystems: Molecular Machinery, Manufacturing, and Computation. John Wiley & Sons, 1992
- **FETTER, A. L ; WALECKA, J. D.** Theoretical Mechanics of Particles and Continua. Dover Publications, 2003
- Basic Capabilities. **FREITAS, R.** Nanomedicine. Landes Bioscience, 1999, vol. I
- **HAPPEL, J. ; BRENNER, H.** Low Reynolds Number Hydrodynamics. The Hague, Kluwer, 1983
- **HILGENFELDT, S ; LOHSE, D.** Response of bubbles to diagnostic ultrasound: a unifying theoretical approach. *European Physic Journal,* 1998
- **HOGG, T.** Using surface-motions for locomotion of microscopic robots in viscous fluids. *J. of Micro-Bio Robotics,* 2014
- **HOGG, T.** Stress-Based Navigation for Microscopic Robots in Viscous Fluids. *Journal of Micro-Bio Robots,* 2018

- **HOGG, T ; FREITAS, R.** Chemical Power for microscopic robots in capillaries. *Nanomedicine: Nanotechnology, Biology and Medicine,* 2010
- **HOGG, T ; FREITAS, R.** Acoustic communication for medical nanorobots. *Nano Communication Networks,* 2012
- **HOGG, T. ; MOSES, M. S. et al.** *Evaluating the Friction of Rotary Joints in Molecular Machines,* 2017
- **LIU, Z. ; LIU, J. Z. et al.** Interlayer binding energy of graphite: A mesoscopic determination from deformation. *Physical Review B,* 2012
- **MATE, C. M.** Tribology on the Small Scale: A Bottom Up Approach to Friction, Lubrication, and Wear. OUP Oxford, 2008
- **PURCELL, E. M.** Life at low Reynolds number. *American J. of Physics,* 1977
- **RAYLEIGH, L.** The principle of similitude. *Nature,* 1915
- **SULLIVAN-SILVA, K. B.** Underwater acoustic scattering from spherical particulates and bubbles. United States Navy, 1989
- **ZHELUDEV, N. L.** The road ahead for meta materials. *Science,* 2010